# EUROPEAN PATENT SPECIFICATION

(11) **EP 3 942 285 B1**
(45) Date of publication and mention of the grant of the patent: **14.05.2025**
(21) Application number: 20774143.0
(22) Date of filing: 20.03.2020
(51) Int. Cl.: G01N 27/403, B82Y 5/00, C12M 1/34, C12Q 1/00, C12Q 1/68, G01N 27/327, G01N 27/416, G01N 33/53, G01N 33/543, G01N 33/569, B82Y 15/00, C12Q 1/6804, C12Q 1/6825

(54) **DIFFERENTIAL SIGNAL BIOSENSING FOR DETECTING AN ANALYTE**
DIFFERENZSIGNALBIOSENSOR ZUM NACHWEIS EINES ANALYTEN
BIODÉTECTION DE SIGNAL DIFFÉRENTIEL POUR DÉTECTER UN ANALYTE

(30) Priority: 20.03.2019 US 201962821219 P
(43) Date of publication of application: 26.01.2022
(73) Proprietor: McMaster University, Hamilton, Ontario L8S 4L8 (CA)
(72) Inventor: SOLEYMANI, Leyla, Oakville, Ontario L6K 3V9 (CA); PANDEY, Richa, Hamilton, Ontario L8S 1H2 (CA); LI, Yingfu, Dundas, Ontario L9H 6Y2 (CA); HOARE, Todd, Ancaster, Ontario L9G 1Z3 (CA); CHANG, Dingran, Hamilton, Ontario L8S 2N6 (CA); LU, Yang, Hamilton, Ontario L8S 3K5 (CA)
(74) Representative: Berggren Oy
(86) International application number: PCT/CA2020/050375
(87) International publication number: WO 2020/186362

(56) References cited:
- WO-A1-2010/003212
- WO-A1-2018/165566
- WO-A1-2018/176042
- WO-A1-2019/099856
- US-A1- 2007 154 909
- US-A1- 2008 185 295
- US-A1- 2014 005 068
- US-A1- 2018 045 668
- SEKER ET AL.: "Electrically Guided DNA Immobilization and Multiplexed DNA Detection with Nanoporous Gold electrodes", NANOMATERIALS, vol. 8, no. 5, 2018, pages 1 - 18, XP055740947
- KELLEY ET AL.: "Ultrasensitive Electrochemical Biomolecular Detection Using Nanostructured Microelectrodes", ACC. CHEM. RES., vol. 47, 2014, pages 2417 - 2425, XP055284840, DOI: 10.1021/ar500130m

## Description

### FIELD

The present application relates to biosensors, and in particular to biosensors and methods for detecting analytes.

### BACKGROUND

Given the rapid emergence of various infectious disease pandemics, point of care (POC) devices have gained significant interest due to their applicability in clinical decision making for rapid, simple, and early screening, diagnosis, and treatment monitoring, for example, for early diagnosis and monitoring in resource limiting settings.

In addition, antibiotic consumption rates are at a record high, contributing significantly to the increased emergence of antibiotic resistance. Advanced diagnostic technologies for identifying infection-causing pathogens enable antibiotics to be prescribed more effectively. Such systems can decrease the sample-to-result time of infectious disease testing, allowing clinicians to intervene and practice more precision medicine. Although molecular diagnostic tests are improving in certain areas, such diagnostic technologies may be costly and more operationally complex to implement at primary health care facilities, particularly in the context of replacing the large volume of cultures performed in the day-to-day diagnosis of common infections such as urinary tract infections (UTIs).

The background herein is included solely to explain the context of the application This is not to be taken as an admission that any of the material referred to was published, known, or part of the common general knowledge as of the priority date.
WO 2018/165566 A1 describes an apparatus for target molecule detection including a sample cell configured to receive a sample, reference, counter, and first and second working electrodes in communication with the sample cell, first and second working electrodes, and a differential amplifier circuit. The first working electrode is coated with a first recognition element that is to interact with a target molecule in the sample, while the second working electrode is not coated with the first recognition element. The first working electrode is configured to measure a first signal responsive to interaction of the first recognition element with the target molecule, while the second working electrode is configured to measure a second signal indicative of background noise from the sample. The differential amplifier circuit configured to generate a modified signal, indicative of an amount of the target molecule present in the sample, that is proportional to a difference between the first and second signals.
WO 2010/003212 A1 describes a biosensing device for detecting a presence of target biomolecules, including at least one working electrode having a systematic array of nano-electrode wires projecting vertically from an electrode pad. The nano-electrode wires all have a same shape and size and are distributed non-randomly over the electrode pad. Biosensor probes are attached to the nano-electrode wire, each including a bioreceptor selected to bind with a complementary target biomolecule to create a binding event; and an electrochemical transducer transducing this binding event into an electrical signal conducted by the corresponding nano-electrode wire.
US 2018/045668 A1 describes apparatuses and methods for analyzing the presence of a target analyte, which can be operated in a multiplexed format to perform various assays of clinical significance.
US 2008/185295 A1 describes sensor devices, methods and kits for detection of biomolecules. The devices employ nanostructured electrode elements including nanotubes, nanoparticles, nanowires, and nanocones. In a particular embodiment, single walled carbon nanotubes disposed in interconnected networks are used as electrodes
US 2014/005068 A1 describes systems and methods for detecting a target analyte in a sample with electrodes, comprising a linker and an antibody attached to the linker, and measuring an electrocatalytic signal changes generated by binding of an analyte in the sample to the antibody. WO 2018/176042 A1 describes an apparatus including a first carbon nanotube array that is patterned to define a first electrode having a first plurality of electrode segments. The apparatus also includes a second carbon nanotube array that is patterned to define a second electrode having a second plurality of electrode segments. The second plurality of electrode segments is interdigitated with the first plurality of electrode segments. The apparatus further includes a biorecognition agent disposed on a surface of the first electrode and disposed on a surface of the second electrode
WO 2019/099856 A1 was filed before the priority date of the present application but published after. The document describes electrochemical aptamer-based (EAB) biosensing devices that provide drift correction and calibration to EAB sensor measurements of biofluid analyte concentrations by disclosing reference sensors that are configured to not interact with a target analyte, but otherwise mirror the performance of active EAB sensors within the expected application parameters of the device. Such reference sensors are configured to allow comparisons with their companion active sensors to track aptamer sensing element dissociation from an electrode surface, temperature-induced effects, redox moiety dissociation, and/or the effects of surface fouling.
SEKER et al., "Electrically Guided DNA Immobilization and Multiplexed DNA Detection with Nanoporous Gold electrodes", Nanomaterials, (20180000), vol. 8, no. 5, pages 1 - 18, describes a study investigate the influence of electrode nanostructure on electrically-guided immobilization of DNA probes for nucleic acid detection in a multiplexed format.
KELLEY et al., "Ultrasensitive Electrochemical Biomolecular Detection Using Nanostructured Microelectrodes", Acc. Chem. Res., (20140000), vol. 47, doi:10.1021/ar500130m, pages 2417 - 2425, describes ultrasensitive electrochemical biomolecular detection using nanostructured microelectrodes

### SUMMARY

The invention is defined in the appended claims.

Other features and advantages of the present application will become apparent from the following detailed description. It should be understood, however, that the detailed description and the specific examples, while indicating embodiments of the application, are given by way of illustration only and the scope of the claims should not be limited by these embodiments, but should be given the broadest interpretation consistent with the description as a whole.

### DRAWINGS

Certain embodiments of the application will now be described in greater detail with reference to the drawings in which:
Figures 1a to 1d show a schematic of target detection using an embodiment of a biosensor.
Figure 2 shows embodiments of electrode chip designs.
Figure 3 shows an embodiment of the operation of an embodiment of a biosensor.
Figure 4 shows an embodiment of an electrochemical performance of embodiments of nanostructured and planar star electrodes: a) H2SO4 scan of the nanostructured and planar electrode for surface area calculation and b) limit of detection of the capture channel on the nanostructured and planar electrode.
Figure 5 shows the sequence and activity of an embodiment of a short DNAzyme and full DNAzyme for *E. coli* detection.
Figure 6 shows an embodiment of a DNAzyme, capture probe and redox-DNA sequences.
Figure 7 shows an embodiment of an electrode-biorecognition element validation for a) release channel by SVW before and after e-DNAzyme immobilization and b) capture channel by CV of pre-probe, post-probe and post-MCH deposition.
Figure 8 shows an embodiment of a SWV of E. coli-spiked buffer on a) release channel and b) capture channel.

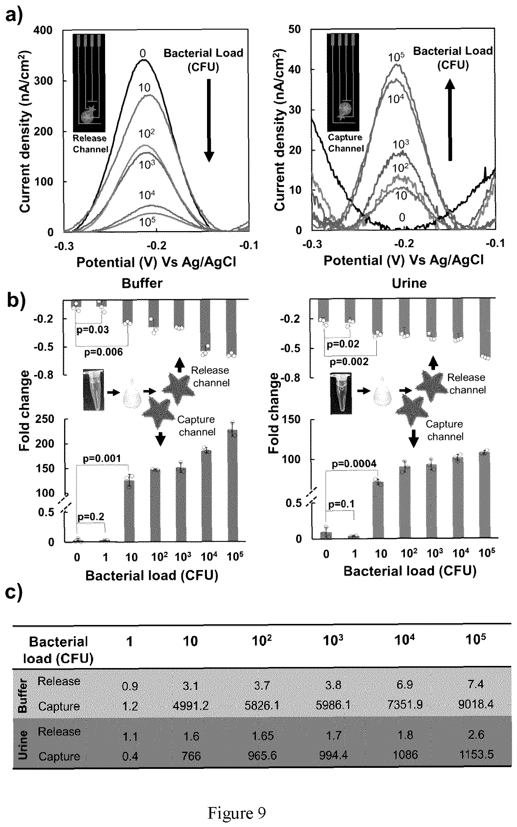

Figure 9 shows an embodiment of bacterial detection using an embodiment of an e-DNAzyme test: a) the electrochemical response of the release (left) and capture (right) channels measured using square wave voltammetry for samples containing different concentrations of *E. coli* spiked in human urine without *E. coli* growth; b) calibration curves of the e-differential chip at varying concentrations of *E. coli* in buffer (left) and spiked in human urine (right) - the top bar graphs demonstrate the response obtained from the release channel, while the bottom graphs demonstrate the capture channel results and the error bars represent the standard deviation from the mean obtained from measurements obtained using three different e-differential chips with the same sample; c) signal-to-blank (S/B) ratios of the release and capture channels for various bacterial loads spiked in buffer (top) and urine (bottom).

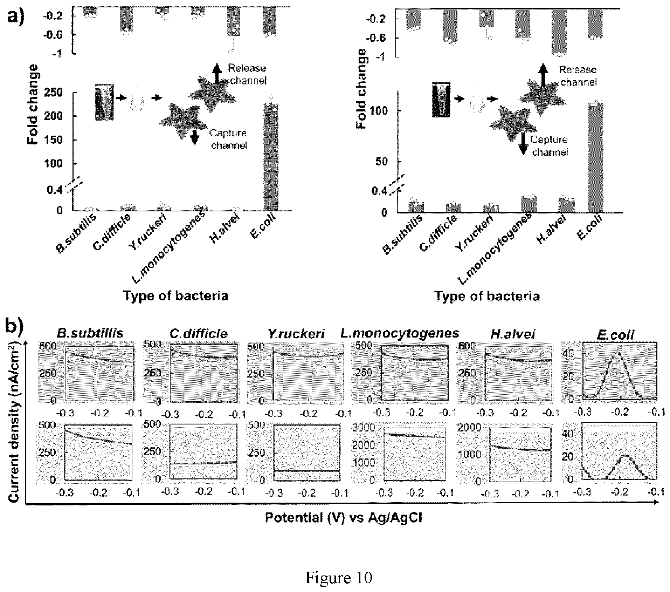

Figure 10 shows evaluation of the specificity of a biosensor in an embodiment: a) Quantification of the cross-reactivity of the chip with different bacteria: B. subtilis (Release channel p= 0.002-0.001; Capture channel p= 0.4-0.05), C. difficile (Release channel p= 0.00003-0.00009; Capture channel p= 0.03-0.01), Y. ruckeri (Release channel p= 0.02-0.03; Capture channel p= 0.1-0.2), L. monocytogenes (Release channel p= 0.01-0.001; Capture channel p= 0.03-0.01), H. alvei (Release channel p=0.0001-0.0006; Capture channel p= 0.4-0.01) and *E. coli* (Release channel p= 0.00002-0.001; Capture channel p= 0.0006-0.000008) suspended in buffer (left) and urine (right). The error bars represent the standard deviation from the mean obtained using three separate devices per sample; b) Square wave voltammetry measurements of the chip modified with E. coli DNAzymes interrogated with various types of bacteria suspended in buffer (top) and urine (bottom). All bacteria are present at 105 CFU/mL.
Figure 11 shows an embodiment of a fluorescent assay performed on an embodiment of *E. coli* specific DNAzymes to extract gel-based cleavage information in urine spiked with *E. coli.*
Figure 12 shows an embodiment of a pre-clinical evaluation of an embodiment of a biosensor for the detection of *E. coli* in urine: a) schematic illustration of the sensing platform (left) and representative square wave voltammograms obtained from E. coli+/culture+, E. coli-/culture+, and E. coli-/culture- samples (right); b) photographs of a subset of urine samples used for analysis (top) and signal fold change measured on the capture channel using 41 clinically-obtained samples (bottom); c) ROC plot (Sensitivity Vs. 1-Specificity) for the pre-clinical data of 41 samples.
Figure 13 shows a culture plate of clinically significant urine sample (> 100 CFU) and clinically insignificant urine sample (1-100 CFU) infected with *E. coli* in an embodiment.
Figure 14 shows results of an embodiment of clinical diagnostic testing for the presence of E. coli in patient urine samples using sensitivity (Se), specificity (Sp), positive predictive value (PPV), negative predictive value (NPV), positive likelihood ratio (PLR), negative likelihood ratio (NLR), accuracy index (AI) and Youden's index (YI) calculations.

### DETAILED DESCRIPTION

### I. Definitions

Unless otherwise indicated, the definitions and embodiments described in this and other sections are intended to be applicable to all embodiments and aspects of the present application herein described for which they are suitable as would be understood by a person skilled in the art. It is also to be understood that the terminology used herein is for the purpose of describing particular aspects only and is not intended to be limiting.

The term "sample" or "test sample" as used herein may refer to any material in which the presence or amount of a target analyte is unknown and can be determined in an assay. The sample may be from any source, for example, any biological (e.g. human or animal samples, including clinical samples), environmental (e.g. water, soil or air) or natural (e.g. plants) source, or from any manufactured or synthetic source (e.g. food or drinks). The sample may be comprised or is suspected of comprising one or more analytes. The sample may be a "biological sample" comprising cellular and non-cellular material, including, but not limited to, tissue samples, urine, blood, serum, other bodily fluids. In an embodiment, the sample comprises urine.

The term "target", "analyte" or "target analyte" as used herein may refer to any agent, including, but not limited to, a small inorganic molecule, small organic molecule, metal ion, biomolecule, toxin, biopolymer (such as a nucleic acid, carbohydrate, lipid, peptide, protein), cell, tissue, microorganism and virus, for which one would like to sense or detect. In an embodiment, the analyte is either isolated from a natural source or is synthetic. The analyte may be a single compound or a class of compounds, such as a class of compounds that share structural or functional features. The term analyte also includes combinations (e.g. mixtures) of compounds or agents such as, but not limited, to combinatorial libraries and samples from an organism or a natural environment. In an embodiment, the analyte comprises a microorganism target.

The term a "microorganism target" as used herein may be a molecule, compound or substance that is present in or on a microorganism or is generated, excreted, secreted or metabolized by a microorganism. In an embodiment, a microorganism target is present in the extracellular matrix of a microorganism. In another embodiment, a microorganism target is present in the intracellular matrix of a microorganism. In another embodiment, a microorganism target comprises a protein, a nucleic acid, a small molecule, extracellular matrix, intracellular matrix, a cell of the microorganism, or any combination thereof. In an embodiment, a microorganism target is a crude or purified extracellular matrix or a crude or purified intracellular matrix. In another embodiment, a microorganism target is specific to a particular species or strain of microorganism.

The term "microorganism" as used herein may refer to a microscopic organism that comprises either a single cell or a cluster of single cells including, but not limited to, bacteria, fungi, archaea, protists, algae, plankton and planarian. In an embodiment, the microorganism is a gram-negative bacterium, for example *Escherichia coli, Salmonella typhimurium, Pseudomonas peli, Brevundimonas diminuta, Hafnia alvei, Yersinia ruckeri, Ochrobactrum grignonese, Achromobacter xylosoxidans, Moraxella osloensis, Acinetobacter lwoffi*, and *Serratia fonticola.* In an embodiment, the microorganism is a gram-positive bacterium, for example *Listeria monocytogenes, Bacillus subtilis, Clostridium difficile, Actinomyces orientalis, Pediococcus acidilactici, Leuconostoc mesenteroides,* and *Lactobacillus planturum.* In another embodiment, the microorganism is a pathogenic bacterium (for example, a bacterium that causes bacterial infection), such as *Escherichia coli* O157:H7, *Listeria monocytogenes, Salmonella typhimurium* or *Clostridium difficile.*

The term "detection probe" as used herein refers to a probe that comprises a recognition moiety and a reporter moiety. The reporter moiety is coupled to a recognition moiety. "Coupled" is used herein to include, for example, covalent binding interactions and/or noncovalent binding interactions (e.g. ionic bonds, Van Der Waals forces, hydrogen bonds, etc.). In an embodiment, the reporter moiety hybridizes to the recognition moiety. In another embodiment, the recognition moiety and the reporter moiety comprise a single molecule.

The term "recognition moiety" as used herein refers to a moiety comprising a molecule (e.g. compound) such as, but not limited to, a DNAzyme, aptamer, antibody, and/or nucleic acid that is able to recognize the presence of an analyte (e.g. bind to the analyte). In an embodiment, the recognition moiety comprises a DNAzyme.

The term "reporter moiety" as used herein refers to a moiety comprising a molecule (e.g. compound) for reporting the presence of an analyte. For example, the moiety is used for transducing the presence of an analyte recognized by the recognition moiety to a detectable signal. In an embodiment, the reporter moiety comprises a molecule modified with a redox, photoelectrochemical, passivating, semi-conductive and/or conductive species. In embodiments, the redox, photoelectrochemical, semi-conductive and/or conductive species decreases a signal on a first working electrode and increases a signal on the second working electrode in the presence of an analyte. In other embodiments, the passivating species increases a signal on a first working electrode and decreases a signal on the second working electrode in the presence of an analyte. In a specific embodiment, the reporter moiety comprises a biopolymer modified with a redox, photoelectrochemical, passivating, semi-conductive, and/or conductive species. In an embodiment, the reporter moiety comprises a nucleic acid sequence modified with a redox, photoelectrochemical, passivating, semi-conductive, and/or conductive species. In an embodiment, the reporter moiety comprises a specific DNA barcode modified with a redox, photoelectrochemical, passivating, semi-conductive, and/or conductive species. Examples of redox species include, for example, methylene blue, methylene blue succinymide, methylene blue maleimide, Atto MB2 maleimide (Sigma Aldrich) and other methylene blue derivatives; 3,7-Bis-[(2-Ammoniumethyl) (methyl)amino]phenothiazin-5-ium trifluoroacetate; 3,7-Bis-(piperazin-4-ium-1-yl)phenothiazin-5-ium trifluoroacetate; 3,7-Bis-[(2-ammoniumethyl)(methyl)amino] phenothiazin-5-ium chloride; and 3,7-Bis-(piperazin-4-ium-1-yl)phenothiazin-5-ium chloride, and/or ferrocene, etc. Examples of photoelectrochemical, semi-conductive and/or conductive species include, for example, photo-active dyes, quantum dots, surface plasmon resonance (SPR) nanoparticles, semi-quantum dots, metal oxides, polymer nanoparticles, and/or metal nanoparticles. Examples of passivating species include insulating compounds/molecules such as insulating polymers and/or insulating metal oxides. These may include particles and beads such as silica beads and/or polystyrene beads.

The term "nucleic acid" as used herein may refer to a polynucleotide, such as deoxyribonucleic acid (DNA), ribonucleic acid (RNA), modified nucleotides and/or nucleotide derivatives, and may be either double stranded (ds) or single stranded (ss). In some embodiments, modified nucleotides may contain one or more modified bases (e.g. tritiated bases and unusual bases such as inosine), modified backbones (e.g. peptide nucleic acid, PNA) and/or other chemically, enzymatically, or metabolically modified forms.

The term "antibody" as used herein may refer to a glycoprotein, or antigen-binding fragments thereof, that has specific binding affinity for an antigen as the target analyte. Antibodies may be monoclonal and/or polyclonal antibodies.

The term "aptamer" as used herein may refer to a short, chemically synthesized nucleic acid molecule or oligonucleotide sequence which can be generated by in vitro selection to fold into specific three-dimensional (3D) structures that bind to a specific analyte with dissociation constants, for example, in the pico- to nano-molar range. Aptamers may be single-stranded DNA, and may include RNA, modified nucleotides and/or nucleotide derivatives. Aptamers may also be naturally occurring RNA aptamers termed "riboswitches".

The term "DNAzyme" as used herein may refer to a nucleic acid molecule or oligonucleotide sequence for specifically recognizing and binding to an analyte. For example, it can catalyze or initiate a reaction in response to specifically recognizing to a target analyte. The DNAzyme may recognize a target analyte and release a reporter moiety upon interaction of the DNAzyme with the target. DNAzymes may be single-stranded DNA, and may include RNA, modified nucleotides and/or nucleotide derivatives. In an embodiment, the DNAzyme catalyzes the cleavage of a particular substrate, for example a nucleic acid sequence comprising one or more ribonucleotides. In an embodiment, the DNAzyme cleaves a single ribonucleotide (RNA) linkage in a nucleic acid sequence wherein the remaining nucleotides are deoxyribonucleotides (DNA). In an embodiment, the DNAzyme cleaves a nucleic acid sequence at a single ribonucleotide linkage thereby producing a nucleic acid cleavage fragment. In an embodiment, the DNAzyme recognizes a microorganism target and cleaves a nucleic acid sequence at a single ribonucleotide linkage upon interaction of the DNAzyme with the target thereby releasing the reporter moiety as the cleavage fragment.

The term "capture probe" as used herein refers to a probe that recognizes and binds to a reporter moiety. In an embodiment, the capture probe comprises a molecule that recognizes and binds (e.g. hybridizes) to a reporter moiety comprising a molecule modified with a redox, photoelectrochemical, passivating, semi-conductive and/or conductive species. In an embodiment, the capture probe comprises a biopolymer. In an embodiment, the capture probe comprises a nucleic acid sequence that hybridizes to the reporter moiety. Examples include ssDNA, ssPNA, dsDNA, ssRNA, and/or dsRNA.

The term "hybridizes" or "hybridization" as used herein refers to the sequence specific non-covalent binding interaction with a complementary nucleic acid sequence.

The term "functionalizing" or "functionalizing on" as used herein may refer to various common approaches for functionalizing a material, which can be classified as mechanical, physical, chemical and biological. Any suitable form of coupling may be utilized (e.g. coating, binding, etc.).

The term "edge" as used herein may be understood to be an area where two plane surfaces meet. The angle between the two plane surfaces may be any suitable range. In particular, in an embodiment, the range is between about 70° and 120°. The area where the two plane surfaces meet may be blunt, in that the intersection between the two planes is not precise or well defined and the transition from one plane to the other may be gradual. A "sharp edge" used herein may be understood to be an edge that has not been rounded. For example, the area where the two plane surfaces meet, in the intersection between the two planes is precise or well defined.

In understanding the scope of the present application, the term "comprising" and its derivatives, as used herein, are intended to be open ended terms that specify the presence of the stated features, elements, components, groups, integers, and/or steps, but do not exclude the presence of other unstated features, elements, components, groups, integers and/or steps. The foregoing also applies to words having similar meanings such as the terms, "including", "having" and their derivatives. The term "consisting" and its derivatives, as used herein, are intended to be closed terms that specify the presence of the stated features, elements, components, groups, integers, and/or steps, but exclude the presence of other unstated features, elements, components, groups, integers and/or steps. The term "consisting essentially of', as used herein, is intended to specify the presence of the stated features, elements, components, groups, integers, and/or steps as well as those that do not materially affect the basic and novel characteristic(s) of features, elements, components, groups, integers, and/or steps.

Terms of degree such as "substantially", "about" and "approximately" as used herein mean a reasonable amount of deviation of the modified term such that the end result is not significantly changed. These terms of degree should be construed as including a deviation of at least 5% of the modified term if this deviation would not negate the meaning of the word it modifies.

As used in this application, the singular forms "a", "an" and "the" include plural references unless the content clearly dictates otherwise.

In embodiments comprising an "additional" or "second" component, the second component as used herein is chemically different from the other components or first component. A "third" component is different from the other, first, and second components, and further enumerated or "additional" components are similarly different.

Although methods and materials similar to those described herein can be used in the practice or testing of this disclosure, suitable methods and materials are described below.

The abbreviation, "e.g." is derived from the Latin exempli gratia and is used herein to indicate a non-limiting example. Thus, the abbreviation "e.g." is synonymous with the term "for example." The word "or" is intended to include "and" unless the context clearly indicates otherwise.

The term "and/or" as used herein means that the listed items are present, or used, individually or in combination. In effect, this term means that "at least one of" or "one or more" of the listed items is used or present.

It will be understood that any component defined herein as being included may be explicitly excluded from the claimed invention by way of proviso or negative limitation.

In addition, all ranges given herein include the end of the ranges and also any intermediate range points, whether explicitly stated or not.

Biosensors can bring together signal transduction and biorecognition elements to analyze biologically-relevant targets. These devices can have a wide range of applications in clinical and agricultural diagnostics, agri-food quality control, environmental monitoring, health monitoring, and pharmaceutical development. Furthermore, these devices can be used in screening. There are several transduction mechanisms that can be used for biosensing (e.g. generating a single signal for each target capture). Using a single signal response in a complex analyte sample may be inefficient due to potential degradation of the biorecognition elements, as well as the interference of background biological materials with the biorecognition and transducer elements, leading to false positive and/or negative results.

**In** an embodiment, a biosensor is provided for detecting an analyte. The biosensor comprises a first and second working electrode, a detection probe functionalized on the first working electrode, a capture probe functionalized on the second working electrode, and a counter electrode. The detection probe functionalized on the first working electrode comprises a reporter moiety linked to a recognition moiety for an analyte. Each working electrode is configured to provide a change in signal if the analyte is present. In a specific embodiment, each of the working electrodes and the counter electrode are configured for current to flow therebetween. In embodiments, the biosensors described herein can allow bacterial analysis to be performed with minimal user intervention in a single incubation step and without, for example, the use of external reagents or labels. In embodiments, the biosensor further comprises a reference electrode. In certain embodiments, the counter electrode acts as both the counter and reference electrode.

**In** embodiments, the biosensors described herein can be used for label-free, and reagent-free detection of analytes. For example, additional labels and/or reagents do not need to be added after the sample is exposed (e.g. introduced, contacted, etc.) to the biosensor. In other embodiments, the biosensors can be used for rapid detection of analytes, including rapid, label-free, and reagent-free detection of analytes.

**In** embodiments of the biosensor, the biosensor has a first working electrode functionalized with biorecognition elements, such as DNAzymes, for the detection of target analytes by generating dual signal responses from a single redox species and another working electrode functionalized with a capture probe for receiving the reporter moiety with the single redox species. The electrodes can allow signals to be gathered from two channels (e.g. signal-on and signal-off channels) to increase the sensitivity, specificity and reliability of the biosensor. The nature of the electrodes can also facilitate the use of a single redox species as part of the reporter moiety to generate a dual response by its release from one electrode and spatial movement to another electrode, where it can be captured. The signal changes of the two electrodes are correlated: analyte capture causes a signal decrease in the first electrode and a signal increase in the second electrode. In embodiments, the geometries of the working electrodes are such that the diffusion length of the reporter moiety comprising the redox species between electrodes can be minimized, and the response time shortened. The multi-electrode biosensor may reduce the signal variation between the electrodes using common on-chip reference and counter electrodes.

**In** an embodiment, binding of the analyte to the detection probe results in delocalization of the reporter moiety from the first to second working electrode and the signal (e.g. current, potential, impedance, etc.) is measured from both the first and second working electrodes. In a further embodiment, the reporter moiety comprises a biopolymer modified with a redox, photoelectrochemical, passivating, semi-conductive and/or conductive species. In an embodiment, the recognition moiety comprises a DNAzyme that cleaves RNA in the presence of the analyte, an aptamer that changes conformation in the presence of the analyte, or an antibody. In a further embodiment, the first working electrode is functionalized with DNAzymes ligated to redox-tagged DNA oligomers and the second working electrode is functionalized with single-stranded DNA (ssDNA) capture probes. When the chip is exposed to the target analyte, the DNAzyme on the first working electrode cleaves at a ribonucleotide site in its sequence to release its redox-DNA reporter segment. This reporter then diffuses towards the second working electrode and hybridizes with the capture probe immobilized on this electrode.

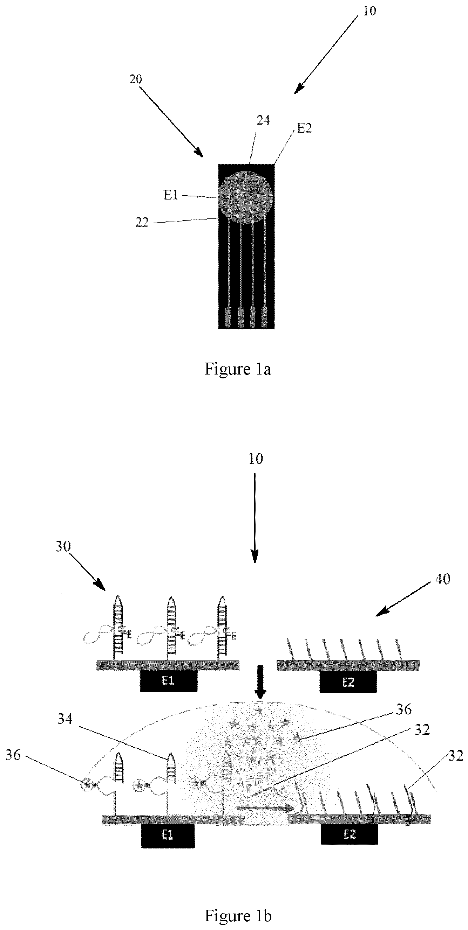

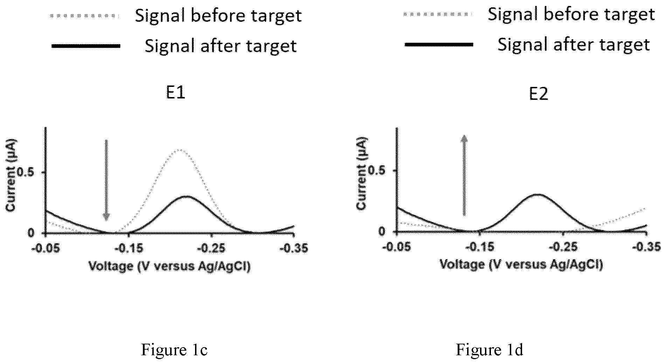

Figures 1a to 1d show an embodiment of a biosensor 10. The biosensor 10 comprising a four-electrode electrochemical chip 20, which is shown in Figure 1a. The four-electrode electrochemical chip 20 comprises an on-chip reference electrode 22, counter electrode 24, a first working electrode E1 and a second working electrode E2 for detecting an analyte. Figure 1b shows the biosensor 10, including the four-electrode electrochemical chip 20, wherein the first working electrode E1 comprises a detection probe 30. The detection probe 30 is functionalized on the first working electrode E1. The detection probe 30 comprises a reporter moiety 32 linked to a recognition moiety 34 for an analyte 36. For example, the recognition moiety 34 comprises biorecognition elements that are ligated to the reporter moiety 32, which comprise redox-tagged DNA oligomers. The second working electrode E2 comprises a capture probe 40. For example, the capture probe 40 comprises single-stranded DNA (ssDNA). When the four-electrode electrochemical chip 20 is exposed to the analyte 36 and the chip is activated (e.g. current applied), the analyte 36 binds to the recognition moiety 34 of the detection probe 30 and the reporter moiety 32 is released and diffuses towards the capture probe 40, where it couples to the capture probe 30. In the absence of the analyte 36, a current peak is generated on E1 (dashed line in Figure 1 c) and no current peak was generated on E2 (dashed line in Figure 1d). In the presence of the analyte 36, the reporter moiety 32 is cleaved from the recognition moiety 34 on E1, and diffuses to the capture probe 40, and binds to the capture probe 40 probe, thereby decreasing the magnitude of the current peak on E1 (solid line in Figure 1c) and increasing it E2 (solid line in Figure 1d) providing a differential signaling approach. In a specific example, when using RNA-cleaving DNAzymes as the detection probe 30 and the chip 20 is exposed to the analyte 36, the DNAzyme on E1 cleaves at a ribonucleotide site in its sequence to release the redox-DNA reporter as the reporter moiety 32. The reporter moiety 32 diffuses towards E2 and hybridizes with the capture probe 40 on E2.

The dual signal approaches can reduce the possibility of false results by providing a secondary signal to validate a primary signal. The dual signal approach is used in fluorescence (Wernette DP, Mead C, Bohn PW, Lu Y. Surface immobilization of catalytic beacons based on ratiometric fluorescent DNAzyme sensors: A systematic study. Langmuir. 2007; 23(18): 9513-9521; Wang J, Wang X, Tang H, et al. A ratiometric magnesium sensor using DNAzyme-templated CdTe quantum dots and Cy5. Sensors Actuators, B Chem. 2018; 272 (October 2017): 146-150; Ling Y, Zhou J, Zhang XF, Wang XH, Li NB, Luo HQ. A ratiometric fluorescent sensor for sensitive detection of UDG using poly(thymine)-templated copper nanoclusters and DAPI with exonuclease III assisted amplification. Sensors Actuators, B Chem. 2019; 286 (January): 46-51; Huang PJJ, Vazin M, Liu J. In vitro selection of a new lanthanide-dependent DNAzyme for ratiometric sensing lanthanides. Anal Chem. 2014;86(19):9993-9999) based sensing, electrochemiluminescence (Wang Y, Shan D, Wu G, et al. A novel "dual-potential" ratiometric electrochemiluminescence DNA sensor based on enhancing and quenching effect by G-quadruplex / hemin and Au-Luminol bifunctional nanoparticles. Biosens Bioelectron. 2018; 106 (January): 64-70; Cheng Y, Huang Y, Lei J, Zhang L, Ju H. Design and biosensing of Mg2+-dependent DNAzyme-triggered ratiometric electrochemiluminescence. Anal Chem. 2014;86(10):5158-5163) based sensing, and electrochemical (Li X, Dou B, Yuan R, Xiang Y. Mismatched catalytic hairpin assembly and ratiometric strategy for highly sensitive electrochemical detection of microRNA from tumor cells. Sensors Actuators, B Chem. 2019;286 (January):191-197; Gao F, Du L, Zhang Y, Tang D, Du Y. Molecular beacon mediated circular strand displacement strategy for constructing a ratiometric electrochemical deoxyribonucleic acid sensor. Anal Chim Acta. 2015; 883:67-73) sensing. The dual-signaling approach can be applied in electrochemical biosensing using two different redox moieties (Gao F, Du L, Zhang Y, Tang D, Du Y. Molecular beacon mediated circular strand displacement strategy for constructing a ratiometric electrochemical deoxyribonucleic acid sensor. Anal Chim Acta. 2015; 883:67-73) in two modes *i.e.* "signal on-off" where the two electrochemical processes respond in an opposite way in the presence of the target (Shakir I, Sarfraz M. Evaluation of Electrochemical Charge Storage Mechanism and Structural Changes in Intertwined MoO3-MWCNTs Composites for Supercapacitor Applications. Electrochim Acta. 2014; 147:380-384), and "signal on/off' where the one signal reports on the presence of the target and the other signal reports on the interference present in the sample (Cheng Y, Huang Y, Lei J, Zhang L, Ju H. Design and biosensing of Mg2+-dependent DNAzyme-triggered ratiometric electrochemiluminescence. Anal Chem. 2014;86(10):5158-5163). However, as it may be difficult to find multiple redox species that are stable under aqueous conditions, a single stable redox species can be used in the embodiments described herein, signal-off and signal-on information can be transmitted as one differential signal by adding or subtracting the two complementary signals to reduce the background and enhance the signal-to-noise ratio.

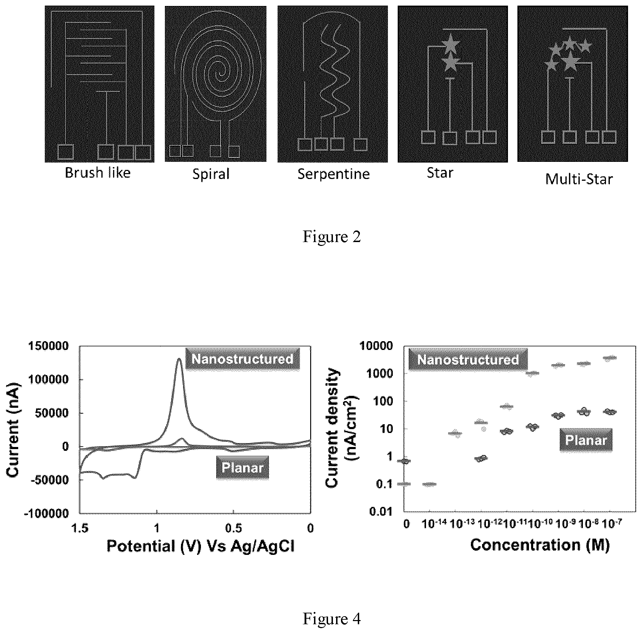

Any suitable electrodes and multiple electrode designs may be used in the biosensors described herein, in particular, in the differential signaling approach. Two or more working electrodes may be used. The electrodes may be any suitable shape, geometry or pattern such as, and without being limited thereto, squares, rectangles, parallelograms, rhombuses, stars, spiral, serpentine, circles, triangles, jigsaw-shaped, etc. In addition, the electrodes may be interdigitated, intermeshed, interleaved or intertwined geometries or patterns such as fingers, dendritic or sawtooth electrode patterns, or indeed any of a number of possibilities that will now become apparent to those skilled in the art, including irregular patterns. Further examples include concentric spiral and serpentine line electrodes, brush electrodes, and intertwined star electrodes and multiple intertwined electrodes such as, and without being limited thereto, multi-star electrodes (Figure 2). Therefore, the biosensor design is also compatible with multiple intertwined electrodes integrated on the same chip for multiplexed analyte detection. In an embodiment, the biosensor provided herein comprises nanostructures with high-aspect ratio on the electrode surface.

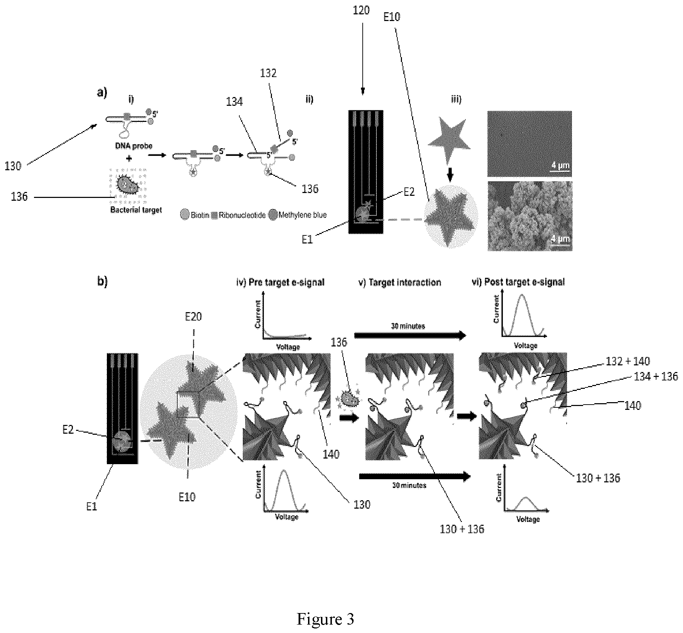

In embodiments, the electrodes are nanostructures. In other embodiments, the electrodes have high-aspect ratios. The high-aspect ratio may be any suitable ratio. Examples of the high-aspect ratio include a vertical-to-lateral ratio ranging from about 2 to about 10, about 3 to about 10, about 4 to about 10, about 5 to about 10, about 6 to about 10, about 7 to about 10. about 8 to about 10, about 2 to about 9, about 3 to about 9, about 4 to about 9, about 5 to about 9, about 6 to about 9, about 7 to about 9, about 8 to about 9, about 2 to about 8, about 3 to about 8, about 4 to about 8, about 5 to about 8, about 6 to about 8, about 7 to about 8, about 2 to about 7, about 3 to about 7, about 4 to about 7, about 5 to about 7, or about 6 to about 7. In embodiments, the electrodes have multiple edges and/or points (see e.g. Figure 3). Such edges and/or points may contribute to high-aspect ratio electrodes (e.g. nanostructured electrodes). The edges and/or points may be sharp. Typical surface areas are about 7 to about 14 times higher in electrodes with such edges and/or points. In certain embodiments, the electrodes described herein have surface areas of about 1.0 cm² to 5.0 cm², about 1.5 cm² to 5.0 cm², about 1.5 cm² to 4.5 cm², about 1.5 cm² to 4.0 cm², about 1.5 cm² to 3.9 cm², about 1.7 cm² to 3.8 cm², or about 1.7 cm² to 3.7 cm², about 1.7 cm² to 3.6 cm², about 1.7 cm² to 3.5 cm², about 1.7 cm² to 3.4 cm², about 1.7 cm² to 3.3 cm², or about 1.7 cm² to 3.2 cm².

In embodiments, the nanostructuring of the electrodes enhanced their limit-of-detection in detecting electroactive oligonucleotides by about 1 to about 3 orders of magnitude compared to their planar counterparts.

Specifically, with respect to intertwined electrodes, intertwining may be used to enhance the proximity of the two or more electrodes and maximize interaction over a smaller area. (Shakir I, Sarfraz M. Evaluation of Electrochemical Charge Storage Mechanism and Structural Changes in Intertwined MoO3-MWCNTs Composites for Supercapacitor Applications. Electrochim. Acta. 2014; 147:380-384; Hou ZQ, Wang ZY, Yang LX, Yang ZG. Nitrogen-doped reduced graphene oxide intertwined with V 2 O 3 nanoflakes as self-supported electrodes for flexible all-solid-state supercapacitors. RSC Adv. 2017; 7(41): 25732-25739; Lee DU, Park W, Park MG, Ismayilov V, Chen Z. Synergistic Bifunctional Catalyst Design based on Perovskite Oxide Nanoparticles and Intertwined Carbon Nanotubes for Rechargeable Zinc-Air Battery Applications. ACS Appl Mater Interfaces. 2015; 7:58.) The electrodes can be intertwined and define a gap therebetween. In an embodiment, the distance between the working electrodes (e.g. E1 and E2) is such that the reporter moiety is capable of transporting (e.g. diffusing and/or active transport) from the detection probe to the capture probe. In embodiments, the electrodes are adjacent to one another and do not contact each other. The gap may be generally constant along the lengths of the electrodes depending on the geometry and shape, for example, such as in spiral and serpentine line electrodes. In embodiments, the gap between the first working electrode and the second working electrode is generally constant along its' length (e.g. serpentine example in Figure 2). In other embodiments, there is a gap between at least a portion of the first working electrode and at least a portion of the second working electrode (e.g. star example in Figure 2), wherein the reporter moiety is capable of transporting (e.g. diffusing and/or active transport) from the detection probe to the capture probe. In embodiments, the gap may be from about 10 µm to about 2 mm, about 10 µm to about 1 mm, about 10 µm to about 500 µm, about 10 µm to about 400 µm, about 10 µm to about 300 µm, about 10 µm to about 200 µm, or about 10 µm to about 100 µm. The intertwined electrodes may have a three-dimensional (3D) nanostructured architecture that increase the sensitivity of the biosensor by 1) increasing the density of the capture probes, 2) improving the accessibility of the capture probes by the target analyte, and/or 3) shortening the diffusion length between the two electrodes. In an embodiment, the intertwined working electrodes are 3D nanostructured electrodes having a high-aspect ratio. In embodiments, the high-aspect ratio is from about 2 to about 10, about 3 to about 10, about 4 to about 10, about 5 to about 10, about 6 to about 10, about 7 to about 10. about 8 to about 10, about 2 to about 9, about 3 to about 9, about 4 to about 9, about 5 to about 9, about 6 to about 9, about 7 to about 9, about 8 to about 9, about 2 to about 8, about 3 to about 8, about 4 to about 8, about 5 to about 8, about 6 to about 8, about 7 to about 8, about 2 to about 7, about 3 to about 7, about 4 to about 7, about 5 to about 7, or about 6 to about 7. The embodiments described herein may improve the reproducibility and reliability of a sensing platform and augment the signal-to-noise ratio that makes a biosensor electrochemically robust in terms of sensitivity, detection limit and Pearson correlation coefficient (Zeng H, Yan Y, Luo X, et al. A review of ratiometric electrochemical sensors: From design schemes to future prospects. Sensors Actuators B Chem. 2018; 274 (July): 501-516) In a further embodiment, the intertwined electrodes may incorporate edges that serve as charge density hot spots for depositing high-aspect ratio 3D nanostructures. In further embodiments, the electrodeposition of such 3D and high-aspect ratio structures on the electrode with solution-penetrating geometry increases the signal magnitude and reduces the mass transport time from E1 to E2.

The electrodes may be constructed of any suitable conductive and/or semi-conductive material(s) comprising a metal, metal alloy, metal oxides, superconductor, semi-conductor, carbon-based materials, conductive polymer, or combinations thereof. The metal may be selected from aluminum (Al), antimony (Sb), bismuth (Bi), boron (B), cadmium (Cd), carbon (C), cerium (Ce), chromium (Cr), cobalt (Co), copper (Cu), dysprosium (Dy), erbium (Er), europium (Eu), gadolinium (Gd), germanium (Ge), gold (Au), graphite (C), hafnium (Hf), holmium (Ho), indium (In), iridium (Ir), iron (Fe), lanthanum (La), lutetium (Lu), magnesium (Mg), manganese (Mn), molybdenum (Mo), neodymium (Nd), nickel (Ni), niobium (Nb), palladium (Pd), platinum (Pt), praseodymium (Pr), rhenium (Re), ruthenium (Ru), samarium (Sm), selenium (Se), scandium (Sc), silver (Ag), silicon (Si), tantalum (Ta), terbium (Tb), thulium (Tm), tin (Sn), titanium (Ti), tungsten (W), vanadium (V), ytterbium (Yb), yttrium (Y), zirconium (Zr) and/or zinc (Zn). Typically, the metals are selected from gold, other noble metals, or combinations thereof. The metal alloy may be selected from the group consisting of aluminum copper (AlCu), aluminum chromium (AlCr), aluminum magnesium (AlMg), aluminum silicon (AlSi), aluminum silver (AlAg), cerium gadolinium (CeGd), cerium samarium (CeSm), chromium silicon (CrSi), cobalt chromium (CoCr), cobalt iron (CoFe), cobalt iron boron (CoFeB), copper cobalt (CuCo), copper gallium (CuGa), copper indium (CuIn), copper nickel (CuNi), copper zirconium (CuZr), hafnium iron (HfFe), iron boron (FeB), iron carbon (FeC), iron manganese (FeMn), iridium manganese (IrMn), iridium rhenium (IrRe), indium tin (InSn), molybdenum silicon (MoSi), nickel aluminum (NiAl), nickel chromium (NiCr), nickel chromium silicon (NiCrSi), nickel iron (NiFe), nickel niobium titanium (NiNbTi), nickel titanium (NiTi), nickel vanadium (NiV), samarium cobalt (SmCo), silver copper (AgCu), silver tin (AgSn), tantalum aluminum (TaAl), terbium dysprosium iron (TbDyFe), terbium iron alloy (TbFe), titanium aluminum (TiAl), titanium nickel (TiNi), titanium chromium (TiCr), tungsten rhenium (WRe), tungsten titanium (WTi), zirconium aluminum (ZrAl), zirconium iron (ZrFe), zirconium nickel (ZrNi), zirconium niobium (ZrNb), zirconium titanium (ZrTi), zirconium yttrium (ZrY), zinc aluminum (ZnAl) and/or zinc magnesium (ZnMg).

The electrodes, and more specifically, the nanostructured electrodes may be made from any suitable method, for example, a seed layer for the nanostructured electrodes may be made by sputter-coating, evaporation, chemical vapor deposition, or a pulsed laser method, ink jet printing. The nanostructures may be made using direct solution based deposition using covalent bonding, electroless or electroplating, electrospinning, template based synthesis, electrophoretic deposition, etching, printing, self-assembly of nanoparticles, etc. In an example, the method comprises applying a patterned mask to a substrate, depositing a conductive material on the mask forming a conductive layer, exposing the conductive layer to pre-conductive material, and using a potentiostatic technique to convert the pre-conductive material to the conductive material, forming the nanostructured electrodes.

Functionalizing of the electrode with the detection probe or capture probe may be done by any suitable techniques, for example, as described in Putzbach, W., & Ronkainen, N. J. (2013). Immobilization techniques in the fabrication of nanomaterial-based electrochemical biosensors: A review. In Sensors (Switzerland) (Vol. 13, Issue 4, pp. 4811-4840). MDPI AG. The method may include binding a detection probe molecule to the surface of the electrode.

It is understood that in the biosensor described herein, the detection probe, the reporter moiety, the recognition moiety, and/or the capture probe may be any suitable species as described above under the definitions section.

In another embodiment, a biosensor comprises a multi-electrode electrochemical chip. The chip has nanostructured intertwined electrodes E1 and E2. A detection probe (e.g. biorecognition elements and redox moieties) is functionalized on E1 and a capture probe is functionalized on E2 (e.g. redox moieties capture probes) for the detection of target analytes. In an embodiment, this biosensor is an integrated four electrode chip including reference and counter electrodes, as well as two working electrodes (E1 = release channel and E2 = capture channel). In a further embodiment, current flows between each of the working electrodes and the counter electrode. In another embodiment, a detection probe comprises a reporter moiety (e.g. redox moiety), which is located on E1 before target introduction and generates a measurable electrochemical current in response to a potential applied to both E1 and E2 electrodes with respect to the reference electrode. Current is still measured as a signal from E1 to provide a quality control checkpoint for the functionality of the assay. Once the target analyte is introduced, the redox moiety moves from E1 to E2, the signal at E1 decreases (signal off), whereas the signal at E2 increases (signal on).

Figure 3 shows another embodiment of the biosensor. The biosensor comprises three components: 1) a detection probe 130 having a recognition moiety 134, which is electroactive biorecognition elements, such as e-DNAzymes, for releasing a reporter moiety 132, which is electroactive DNA barcodes, in the presence of an analyte 136, which is specific bacterial targets (Figure 3 a), i); 2) electrochemical differential chips 120 (e.g. a four-electrode chip) with a first working electrode E1 and a second working electrode E2. The first working electrode E1 is a release channel E10 and the second working electrode is a capture channel E20. The two-channel design for translating e-DNAzyme cleavage on the release channel E10 to barcode binding on the capture channel E20 using a single redox moiety (e.g. methylene blue). This can suppress the effect of non-specific e-DNAzyme cleavage that typically reduces signal-to-blank ratios in biological samples (Figure 3 a), ii); and 3) three-dimensional (3D) nanostructured electrochemical E1 and E2 channels, for example, a dendritic morphology, to increase the capture and hybridization efficiency of the DNA barcodes to improve sensitivity (Figure 3 a), iii). The capture and release channels E10 and E20, can be nanostructured using electrodeposition. The capture and release channels E10 and E20 have multiple edges (e.g. sharp) which can result in diffusion-limited growth and thus the deposition of a dense layer of nanostructures organized in high-aspect ratio architectures (Figure 3 a), iii). In the absence of the analyte 136, a large current peak is generated on the release channel E10 due to the reduction of the single redox moiety (e.g. methylene blue) (see bottom graph in Figure 3 b) iv)) but no current peak was generated on the capture channel E20 (see top graph in Figure 3 b) iv)). In the presence of the analyte 136, the analyte 136 binds to the detection probe 130 (see Figure 3 b) v)), the reporter moiety 132 (e.g. electroactive barcode) is cleaved from the detection probe 130 (e.g. DNAzyme) on the release channel E10, diffused to the capture channel E20, and hybridize with the capture probe 140 (e.g. ssDNA probe) on the capture channel E20 (see Figure 3 b) vi)), thereby decreasing the magnitude of the current peak on the release channel E10 (see bottom graph in Figure 3 b) vi)) and increasing it on the capture channel (see top graph in Figure 3 b), vi)). In embodiments, the biosensors can allow bacterial analysis to be performed with minimal user intervention in a single incubation step and without the use of external reagents or labels. In embodiments, the biosensors described herein can be used for label-free, and reagent-free detection of analytes. In other embodiments, the biosensors can be used for rapid detection of analytes, including rapid, label-free, and reagent-free detection of analytes.

In embodiments, the biosensors, kits and methods of use described herein can be used for detecting any suitable analyte, such as, and without being limited thereto, a wide range of small molecule, protein and nucleic acid analytes, including infection-causing pathogens in point-of-care diagnostics and health monitoring. In a further embodiment, the biosensor and/or method are used to detect E. coli in buffer and urine test samples. In embodiments, the biosensors enable an ultra-sensitivity (10 CFU) and high specificity against a panel of bacteria.

In an embodiment of the kit, the kit comprises a biosensor described herein and instructions for use.

The biosensors described herein can be used in any suitable device, including a hand-held device and/or diagnostic device. For example, the device may resemble the glucose monitor in terms of ease-of-use, portability, cost, and/or amplification-free bacterial detection based, for example, on an electrochemical readout. In an embodiment, the device may be a handheld electrical reader comprises the biosensor (e.g. self-contained e-differential chip).

In further embodiments, the biosensors described herein may be used in a handheld screening and/or diagnostic device that performs benchtop testing and generates test results in less than about 30 minutes, less than about 20 minutes, or less than about 15 minutes, for example, while matching the clinical sensitivity and specificity offered by comparative tests (e.g. urine cultures that require days to complete). In embodiments, the biosensor may be used without the need for sample pre-treatment, target labeling, and/or amplification.

Urinary tract infections, despite being the second most common bacterial infectious disease, lacks the developments towards affirmative early diagnosis albeit the challenges of antibiotic resistance associated with it. The existing in-patient or outpatient approaches to urine analysis suffer from false positive results and sample-to-answer time of 1-2 days causing delays in the disease treatment, and an increased cost of care. The devices described herein may increase the accuracy and decrease the timeline for diagnosis.

**In** an embodiment of a method for determining the presence of an analyte in a sample. The biosensor described herein is exposed to the sample. The reporter moiety delocalizes from the first working electrode to the second working electrode in the presence of the analyte, providing a change in signal (e.g. current, potential, impedance, etc.) at each of the first working electrode and the second working electrode. The change in signal indicates the presence of the analyte. **In** various embodiments, the change in signal for the first working electrode is from about 0.1 to about 0.6 fold decrease and the change in signal for the second working electrode is from about 50 to about 200 fold increase or the change in signal for the first working electrode is from about 50 to about 200 fold increase and the change in signal for the second working electrode is from about 0.1 to about 0.6 fold decrease. **In** embodiments, the signal is current. With respect to the fold decrease, the ranges may be from about 0.1 to about 0.6 fold decrease, about 0.1 to about 0.5 fold decrease, about 0.1 to about 0.4 fold decrease, about 0.1 to about 0.3 fold decrease, about 0.1 to about 0.2 fold decrease, about 0.2 to about 0.6 fold decrease, about 0.3 to about 0.6 fold decrease, about 0.4 to about 0.6 fold decrease, or about 0.5 to about 0.6 fold decrease. With respect to the fold increase, the ranges may be from about 50 to about 200 fold increase, from about 50 to about 150 fold increase, from about 50 to about 100 fold increase, from about 50 to about 90 fold increase, from about 50 to about 80 fold increase, from about 50 to about 70 fold increase, from about 50 to about 60 fold increase, from about 60 to about 200 fold increase, from about 70 to about 200 fold increase, from about 80 to about 200 fold increase, from about 90 to about 200 fold increase, from about 100 to about 200 fold increase, from about 125 to about 200 fold increase, from about 150 to about 200 fold increase, or from about 175 to about 200 fold increase.

In embodiments, if there is no measurable signal change, the analyte is not present in the sample. **In** another embodiment, if the change in signal from the first working electrode is a decrease in signal and the change in signal from the second working electrode is an increase in signal, the analyte is present. **In** another embodiment, if the change in signal from the first working electrode is an increase in signal and the change in signal from the second working electrode is a decrease in signal, the analyte is present.

**In** embodiments, if no measurable current change, the analyte is not present in the sample. **In** another embodiment, the change in current from the first working electrode is a decrease in current and the change in current from the second working electrode is an increase in current, the analyte is present. In another embodiment, if the change in current from the first working electrode is an increase in current and the change in current from the second working electrode is a decrease in current, the analyte is present.

**In** embodiments, the biosensor described herein is exposed to the sample under conditions to delocalize the reporter moiety from the first working electrode to the second working electrode in the presence of the analyte, providing the change in signal.

In the present invention the signal is measured from each of the working electrodes. For example, the current is measured from each of the working electrodes.

**In** other specific embodiments of the method described herein, prior to exposing the biosensor to the sample, an initial signal is measured at each of the first and second working electrodes. After exposing the biosensor to the analyte, a final signal is measured at each of the first and second working electrodes. A change in signal between the initial signal and the final signal of the first working electrode and a change in signal between the initial signal and the final signal of the second working electrode, indicates the presence of the analyte in the sample.

**In** other specific embodiments of the method described herein, prior to exposing the biosensor to the sample, an initial current is measured at each of the first and second working electrodes. After exposing the biosensor to the analyte, a final current is measured at each of the first and second working electrodes. A change in current between the initial current and the final current of the first working electrode and a change in current between the initial current and the final current of the second working electrode, indicates the presence of the analyte in the sample.

The above disclosure generally describes the present invention. A more complete understanding can be obtained by reference to the following specific Examples. These Examples are described solely for purposes of illustration and are not intended to limit the scope of the invention.

Although specific terms have been employed herein, such terms are intended in a descriptive sense and not for purposes of limitation.

### EXAMPLES

### Example 1. Preparation and Characterization of an Electrochemical Biosensor

A four-electrode electrochemical chip was fabricated on pre-stressed polystyrene (PS) sheets (Graphix Shrink Film, Graphix). The PS was cleaned with ethanol and DI water, after which a vinyl mask (FDC 4304, FDC graphic films) was applied onto the sheet. The vinyl mask was then cut into the star-shaped working electrode pattern (as defined by Adobe Illustrator) using a Robo Pro CE5000- 40-CRP cutter (Graphtec America Inc.). The vinyl was then peeled off, and a 100 nm gold (Au) layer was sputtered on top using DC sputtering (MagSput^{™}, Torr International). The chip comprises two star-shaped working electrodes (WEs; release channel, E1 and capture channel, E2) and both counter (CE) and reference (RE) electrodes. This chip with the conductive layer served as a seed layer for the nucleation and growth of high-aspect ratio nanostructures using an electrodeposition technique. A 10 mM gold chloride (HAuCl₄) in 5 mM HCl solution was bubbled with N₂, before use, for about 10 minutes. The conductive layer containing the electrodes was dipped in this solution and gold nanostructures were electrodeposited on the two working electrodes by applying a static potential of about -0.6V (anodic negative) for about 600 s using a potentiostatic technique to reduce the gold ions. Both E1 and E2 were deposited with gold (Au) nanostructures. The resulting chip comprised two nanostructured star-shaped working electrodes (WE; release channel, E1 and capture channel, E2) and both counter (CE) and reference (RE) electrodes. The nanostructuring of the electrodes enhanced their limit-of-detection in detecting electroactive oligonucleotides by three orders of magnitudes compared to their planar counterparts (Figure 4).

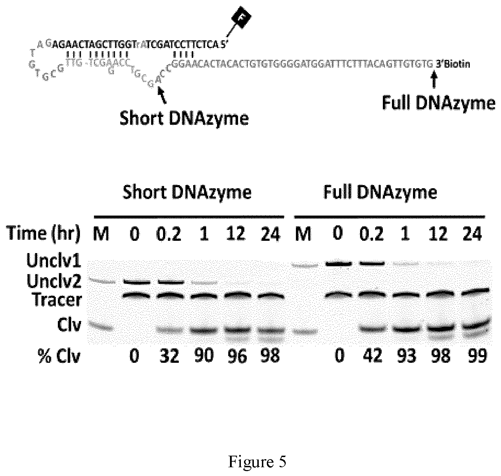

A truncated version of the RNA-cleaving fluorescent DNAzyme for *E. coli* detection (RFD-EC1) as described in Ali *et al.* (Ali MM, Aguirre SD, Lazim H, Li Y. Fluorogenic DNAzyme Probes as Bacterial Indicators. Angew. Chem. Int. Ed. 2011; 50:3751-3754) was used. The truncated DNAzyme has a secondary structure for better release of the cleaved redox-tagged DNA segment. The shorter sequence increased the loading capacity on the electrode and reduced cost. The truncated DNAzyme had similar activity and specificity to the RFD-EC1 (Figure 5). Further modification included adding biotin on the 3'-end for immobilization on the electrode and a methylene blue on the 5'-end as the redox species to generate the signal. The sequence was also extended on the 5'-end by 11 nucleotides to improve the hybridization efficiency between the redox-tagged DNA reporter (i.e. the cleavage product) and the capture probes on E2. Furthermore, the fluorophore and quencher from the original RFD-EC1 were removed.

The e-DNAzymes were prepared by T4 DNA ligase-mediated DNA ligation of the biotinylated DNAzyme sequence (BD) and labeled DNA reporter (DR) in the presence of a ligation template (LT) as the template to complete the biotinylated DNAzyme reporter (BDR). The methylene blue labeled DNA was purchased from Biosearch Inc, and all other DNA oligonucleotides (including the thiolated probe, TP) were purchased from Integrated DNA Technologies. All DNA oligonucleotides were purified via standard 10% denaturing (7 M urea) polyacrylamide gel electrophoresis (dPAGE), with their concentrations determined spectroscopically. The sequences and functions of the synthetic oligonucleotides used herein are provided in Table 1. ATP, T4 DNA ligase, T4 polynucleotide kinase (PNK), and T4 DNA ligase, along with their respective buffers, were purchased from Thermo Scientific. Water used in the experiments (ddH₂O) was purified with a Milli-Q Synthesis A10 water-purification system. All other chemicals were purchased from either Sigma-Aldrich or Bioshop Canada and were used without further purification.

**Table 1. Sequences of, and modifications to, the oligonucleotides**

| Name | Labels | Sequence (5'-3') | Note |
|---|---|---|---|
| BDR | 5'-Methylene blue; R = riboA; 3'-Biotin | MBTTTTTTGTGTGACTCTTCCTAG CTRTGGTTCGATCAAGAGATGTGC GTCTTGATCGAGACCTGCGACCGT TTTTTTTTTBio | *E. coli* DNAzyme probe (Ec-DP) |
| BD | 3'-Biotin | GATGTGCGTCTTGATCGAGACCTG CGACCGTTTTTTTTTTBio | Biotinylated part of Ec-DP |
| DR | 5'-Methylene blue; R = riboA | MBTTTTTGTGTGACTCTTCCTAG CTRTGGTTCGATCAAGA | MB tagged Ec-DP |
| TP | 3'-Thiol | TAGCTAGGAAGAGTCACACA | Capture DNA probe |
| LT | None | CAAGACGCACATCTCTTGATCGAA CC | Ligation template |

1 nmol of BD was phosphorylated in 100 µL of 1× PNK buffer A containing 2 mM ATP (final concentration) with 20 U (units) of PNK enzyme at 37 °C for 40 min. The reaction was quenched by heating the mixture at 90 °C for 5 min. Then, an equal number (1 nmol) of MS and LT were added to the reaction mixture. The mixture was then heated at 90 °C for 1 min before being cooled over 15 min until it reached room temperature. Once the mixture had cooled, 20 µL of 10× DNA ligase buffer and 20 U of T4 DNA Ligase was added, and the final volume was adjusted to 200 µL by adding ddH₂O. After incubation at room temperature for 2 h, the DNA molecules were isolated by ethanol precipitation and the ligated DNA molecules were purified via 10% dPAGE. After being dissolved in ddH₂O, the DNAzyme concentration was measured using a Nanovue and the DNAzyme solutions were stored at -20 °C until use. Fluorophore-labeled (using FAM in place of methylene blue) DNAzyme was also prepared by T4 DNA ligase-mediated DNA ligation of BD and DR in the presence of LT as the template using the same ligation protocol.

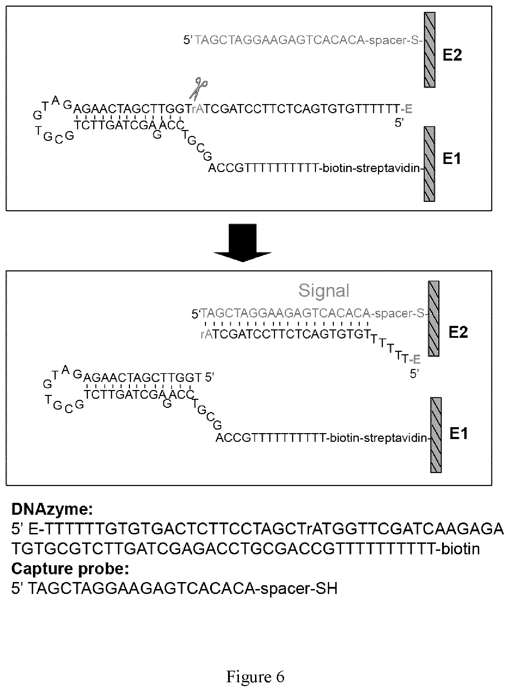

The e-differential chips were rinsed in isopropanol and ddH₂O. This was followed by electrochemical cleaning using cyclic voltammetry (CV) in 0.1 M H₂SO₄ (0 V-1.5 V, 100 mV/s, 40 cycles). After cleaning of the e-differential chips, the release and capture channels were functionalized with their respective biorecognition elements. To functionalize the release channel (E1), 50 mM cysteamine hydrochloride was drop-deposited for 20 hours followed by 10% glutaraldehyde (pH=7) for 12 hours in the dark at room temperature. 1 µM streptavidin was then deposited on the cysteamine-glutaraldehyde functionalized release channel for 4 hours at 4°C, followed by washing in buffer and the deposition of 2 µM of biotinylated DNAzyme reporter (BDR; sequence in Figure 6) for 30 minutes at room temperature. To functionalize the capture channel (E2), a 1.5 µM thiolated probe (TP; sequence in Figure 6) was reduced with 150 µM tris(2-carboxyethyl) phosphine (TCEP) for two hours in the dark at room temperature and then deposited on the capture channel for 18 hours. Following, a 100mM 6-mercaptohexanol (MCH) solution was used to backfill the surface for 20 minutes in the dark at room temperature.

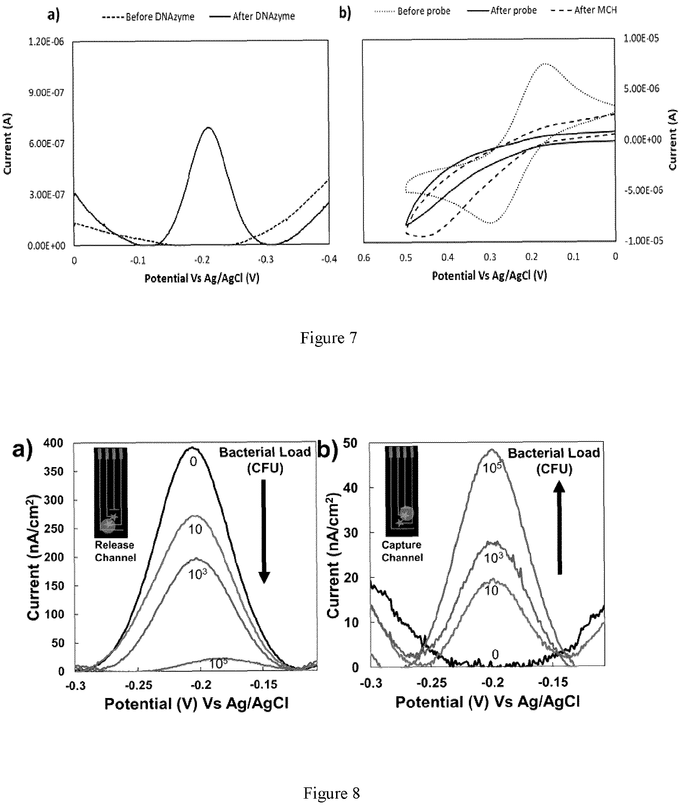

The electrochemical surface area was calculated by performing a cyclic voltammetry scan from 0 to 1.5V (cathodic positive) in 0.1 M H₂SO₄ using a scan rate of 50 mV/s. The area under the reduction peak was integrated to calculate the electrochemical charge involved in the redox process, which was subsequently divided by the surface charge density involved in forming a monolayer of AuOₓ (386 µC/cm²). The immobilization of BDR on release channel E1 was characterized by square wave voltammetry (SWV) over a voltage range of 0 V to -0.6 V (anodic negative) before and after the DNAzyme deposition steps in 25 mM PBS and 25 mM NaCl buffer (25:25 buffer). As the DNAzyme has the redox species attached (before target addition), before the incubation there was no peak whereas after the incubation the peak emerges. Thus, the methylene blue moieties on the chemically immobilized e-DNAzymes participated in charge transfer with the release channel and resulted in a current peak in SWVs, validating DNAzyme immobilization on the electrode surface (Figure 7(a)). The immobilization of the thiol terminated probe (TP) on the capture channel E2 was investigated using a cyclic voltammetry (CV) scan from 0 V to 0.5 V (anodic positive) with a scan rate of 50 mV/s in 2 mM ferrocyanide (FOCN) solution. A flat band was observed after probe indicating the blockage of the redox of Fe⁺³/Fe⁺⁴ ions in the solution. After MCH deposition the reduction peaks appears again confirming the optimum layout of the probe for charge transfer. Thus, the capture channel was functionalized with ssDNA probes with a sequence complementary to the electroactive barcode of the release channel (Figure 7(b)).

### Example 2. Detection of the Presence of Bacteria in Buffer and Urine Samples

To assess the ability of the e-DNAzyme test to detect clinically relevant amounts of bacteria, various known concentrations of *E. coli* were tested in buffer and spiked in urine. *E. coli* was chosen as the target due to its predominance in urinary tract infections (UTIs), which is one of the most prevalent bacterial infections in clinical practice; however, the e-DNAzyme strategy could in principle be applied to any relevant bacteria.

For testing the assay in buffer or urine samples, pathogenic samples comprised a crude intracellular mixture (CIM) of *E. coli* (test sample containing target analyte) or *B. subtilis, Y. ruckeri, C. difficile, H. alvei* or *L. monocytogenes* bacteria (control samples). *Escherichia coli* K12 (*E. coli* K12; MG1655) was used herein. To measure the colony-forming units (CFU/mL) of *E. coli* cells, a single colony freshly grown on Luria Broth (LB) agar plate inoculated into 2 mL of LB and grown for 14 h at 37 °C with continuous shaking at 250 rpm. Following incubation, a 10-fold serial dilution of bacterial culture was conducted, and 100 µL of the diluted solutions were then spread onto LB agar plates (in triplicate) and incubated at 37°C for 16 h. Finally, the colonies were counted and averaged to obtain the number of CFU/mL. For the preparation of bacterial CIM samples for testing, 1 mL of each dilution was centrifuged at 11,000 g for 5 min at 4°C. The clear supernatant was then discarded, and the cell pellet was resuspended in 100 µL of ddH₂O and heated at 65 °C for 10 min to release the DNAzyme-activating target. The heat-treated cell suspension was then vortexed to dissolve the cell pellet completely and stored at -20 °C. Different concentrations of bacterial CIM were spiked into both the 25:25 buffer and healthy human urine samples. After chip fabrication and functionalization, 10 µL of bacteria-doped samples were incubated on top of the chip for 30 minutes at 37°C. The signal generated with *E. coli* was compared to a panel of the five control samples containing CIM from the other bacteria (*B. subtilis, Y. ruckeri, C. difficile, H. alvei* and *L. monocytogenes*) to assess the specificity toward *E. coli.* A small volume (10 µL) of these samples were dropped on the two working electrodes (E1 and E2) for 30 min at 37 °C. The signal quantification was performed using SWV over a voltage range of 0 V to -0.6 V (anodic negative) in 25:25 buffer and urine.

*E. coli* samples having a bacterial load of 0-10⁵ CFU were introduced on the biosensor and the current generated on the release and capture channels were measured using SWV (Figure 8 and Figure 9(a)). The signal changes induced on each channel were quantified by calculating the difference between the current measured before and after introducing the bacterial load (Figure 9(b)). This improved the reliability of the sensor by eliminating false results and enhanced the performance by reducing the signal-to-noise ratio. After incubating the test samples on the biosensor, target binding the DNAzyme caused the ribonucleotide cleavage that released the redox-DNA reporter, leading to a decrease in the signal from E1. An increase in the signal is observed from E2 due to the capture of the redox-DNA. Both the buffer and the urine blank measurements showed a decrease in the E1 signal, which was deducted from the signal obtained from test samples to remove the contribution of background signal from the blank samples. The signal changes on the release channel were lower than one-fold (100%); however, signal changes larger than 50-fold (5000 %) were observed on the capture channel. Starting at a bacterial load of 10 CFU, the biosensor was able to distinguish between samples with and without *E. coli* on both channels. The high signal-to-blank ratio of the designed assay achieved by the four-electrode design still successfully detected the presence of as few as 10 CFU of bacteria in urine (Figure 9(c)).

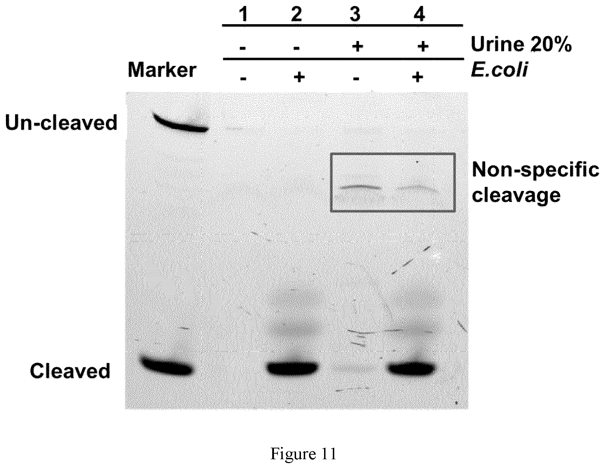

The e-DNAzyme test could sensitively detect *E. coli* in both buffer and urine, the specificity of this assay was assessed against a panel of gram-negative and gram-positive bacterial targets: *B. subtilis, Y. ruckeri, C. difficile, H. alvei* and *L. monocytogenes* (Figure 10).The pathogen-containing control samples with non-target bacteria caused a decrease in the E1 signal due to non-specific cleavage of DNAzyme. However, no significant signal increase is observed from E2 given that the non-specific DNAzyme cleavage does not release a redox-DNA reporter that is complementary to the probe immobilized on E2. The signal from urine samples show a larger decrease in E1 signal than from the buffer samples due to the presence of non-specific exo/endonuclease activity in urine. Therefore, specific identification of *E. coli* relative to other bacteria in urine or buffer was possible using the release channel signal and the capture channel, which allowed precise identification of the *E. coli* sample. Samples containing *E. coli* in buffer and urine resulted in signal changes of at least 200 and 100-folds respectively, while samples containing bacteria other than *E. coli* yielded signal changes of less than 0.4-fold in both urine and buffer. The high specificity of this assay is rooted in the use of two biorecognition events (specific cleavage on release channel and efficient hybridization on capture channel), which reduced the generation of signals through non-specific cleavage. In particular, while non-specific cleavage of DNAzymes by DNA/RNA cleaving enzymes is increased in biological materials, these non-specific interactions do not necessarily result in cleaved segments complementary to the full sequence of the ssDNA probe on the capture channel (Figure 11). The generation of the redox current on the capture electrode can be dependent on proximity between the electrode and the methylene blue molecule, which can be dependent on the length and sequence of the redox barcode.

### Example 3. Detection of Bacteria in Clinical Human Urine Samples

To demonstrate the utility of the e-DNAzyme test in a practical clinical context, the RCDs specific to *Escherichia coli* were used to assess the operation of the e-DNAzyme test towards the identification of this pathogen in UTIs using clinical human urine samples that were bacteria negative, bacteria positive but *E. coli* negative, and *E. coli* positive.

A set of 41 urine samples including 20 clinically-significant *E. coli-only,* 10 clinically-insignificant *E. coli*-only, 2 *K. pneumonie-only, 2 E. faecalis*-only, *1 S. aureus-only,* and 1 *P. mirabilis-only* urine samples, and 5 culture-negative (i.e. no bacterial growth) urine samples were obtained from Hamilton General Hospital. The urine samples were heated at 55°C for 15 minutes followed by centrifugation at 11,000 × g for 5 minutes. A 10 µL of the suspension of the urine was then incubated on the chip for 30 minutes at 37°C, after which SWV over a voltage range of 0 V to -0.6 V (anodic negative) was conducted in 25:25 buffer. Clinical sensitivity was calculated by dividing "True Positive" results from a sum of "True Positive" and "False Negative" results, while clinical specificity was calculated by dividing "True Negative" results from a sum of "True Negative" and "False Positive" results.

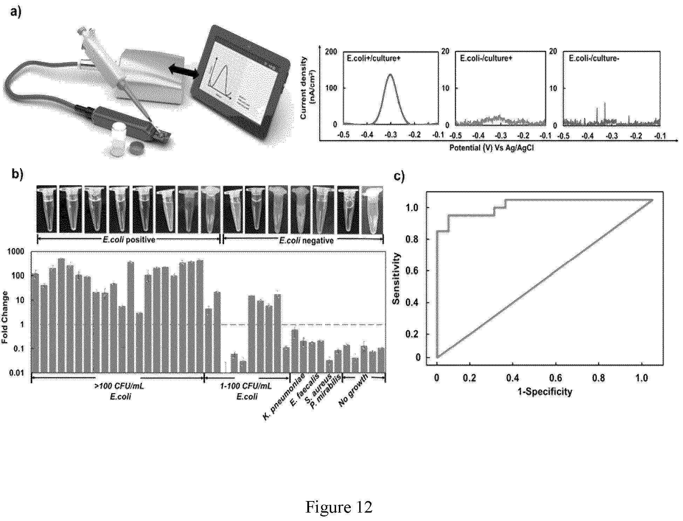

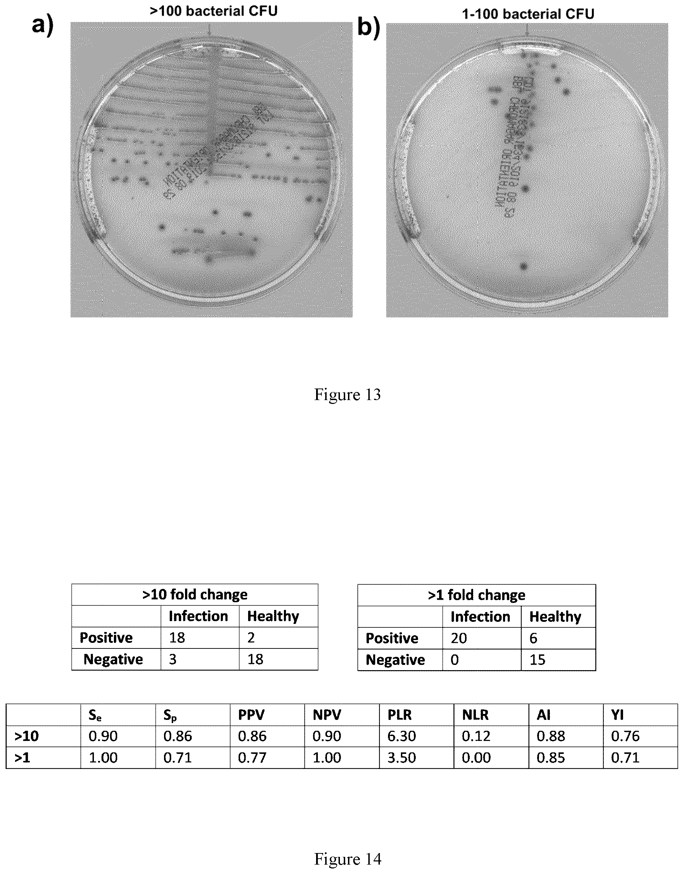

The disclosed biosensor was used to analyze the 41 urine samples collected from patients who were suspected of having UTIs due to the presence of clinical symptoms. A handheld electrical reader comprises the self-contained e-differential chip to demonstrate the potential of the e-DNAzyme test for point-of-care analysis (Figure 12(a left)). A set of randomized samples belonging to the following categories was analyzed: culture-, culture+/*E*. *coli-,* and culture+/¬*E*. *coli+.* These sample categories resulted in specific redox signatures: the culture- samples showed no peak, the culture+/*E*. *coli-* samples exhibited a peak with a low signal-to-noise ratio and culture+/*E*. *coli+* samples resulted in a distinct peak with a high signal-to-noise-ratio (Figure 12(a), right). Defining the detection threshold as a one-fold increase in signal on the capture channel successfully distinguished between *E. coli+* (*E. coli*+/culture+) *and E. coli- (E. coli*+/culture-, *E. coli*-/culture+, *E.*coli-/culture-) samples (Figure 12b). For concentrations >100 CFU/mL (Figure 13(a)), 100% of the *E. coli+* samples were correctly categorized; within the 1-100 CFU/mL range (Figure 13(b)), 60% of the *E. coli+* samples were correctly categorized. Considering that bacterial concentrations lower than 10⁴ CFU/mL are considered clinically insignificant, a clinical sensitivity of up to 100% and specificity of up to 86% was achieved (Figure 14). Additionally, in the *E. coli-* samples, 100% of these samples were successfully categorized as negative. The significant variation in the measured signal change observed in the *E. coli+* samples, even amongst those in the >100 CFU/mL range, can be attributed to the actual variations in target concentration, sample constituent heterogeneity (Figure 12(b)-top), and the presence of blood, casts, proteins, and epithelial cells in the samples. A Receiver operating characteristic (ROC) plot was constructed to illustrate the diagnostic ability of the test for its application in clinical settings (Figure 12(c)). An area under the curve (AUC) of 0.96 demonstrates a reliable diagnostic test for clinical studies.

The biosensor allowed the analysis of clinical urine samples using a handheld reader without the need for sample pre-treatment, target labeling, enrichment or amplification and readout reagents within 30 minutes while matching the clinical sensitivity and specificity offered by urine cultures that require days to complete.

The design and fabrication of a highly specific and sensitive electrochemical assay based on the integration of RNA-cleaving DNAzyme technology, differential electrochemical signal readout, and nanostructured electrodes for rapidly identifying bacteria without time-consuming growth cultures is disclosed herein. Differential electrochemical signaling is made possible due to the programmable nature of electroactive RNA-cleaving DNAzymes combined with a two-channel electrochemical chip. Each of the building blocks of the e-DNAzyme assay contributes to enhanced sensitivity, specificity, or speed of operation. This system can be engineered to target and identify multiple bacteria in parallel for performing rapid, multiplexed, and reagent-free bacterial analysis.

While the present application has been described with reference to examples, it is to be understood that the scope of the claims should not be limited by the embodiments set forth in the examples, but should be given the broadest interpretation of the wording of the appended claims.

## Claims

1. A biosensor (10) for detecting an analyte (36) comprising:
a) a first (E1) and second (E2) working electrode;
b) a detection probe (30) functionalized on the first working electrode, the detection probe comprising a reporter moiety (32) coupled to a recognition moiety (34) for an analyte, wherein the detection probe is adapted for delocalizing the reporter moiety from the first working electrode to the second working electrode when the analyte couples to the recognition moiety;
c) a capture probe (40) functionalized on the second working electrode, wherein the capture probe is for recognizing and coupling to the reporter moiety; and
d) a counter electrode (24);
wherein each working electrode is configured to provide a change in signal if the analyte is present.

2. The biosensor of claim 1, wherein:
the biosensor further comprises a reference electrode (22); or
the counter electrode is both a counter electrode and a reference electrode.

3. The biosensor of claim 1 or 2, wherein one or more of:
the first (E1) and second (E2) working electrodes are selected from concentric spiral electrodes, serpentine line electrodes, brush electrodes, intertwined star electrodes, and/or multiple intertwined electrodes;
the first working electrode and the second working electrode are in proximity to each other such that the reporter moiety is capable of transporting from the detection probe to the capture probe;
the working electrodes are adjacent to one another; and
there is a gap between at least a portion of the first working electrode and at least a portion of the second working electrode, wherein the reporter moiety is capable of transporting from the detection probe to the capture probe; optionally, the gap is from about 10 µm to about 2 mm, about 10 µm to about 1 mm, about 10 µm to about 500 µm, about 10 µm to about 400 µm, about 10 µm to about 300 µm, about 10 µm to about 200 µm, or about 10 µm to about 100 µm.

4. The biosensor of any one of claims 1 to 3, wherein one or more of:
at least one of the first (E1) and second (E2) working electrodes is a nanostructured electrode;
at least one of the first and second working electrodes has edges; optionally, at least one of the first and second working electrodes has sharp edges and/or points;
at least one of the first and second working electrodes has a high-aspect ratio; optionally, the high-aspect ratio is from about 2 to about 10, about 3 to about 10, about 4 to about 10, about 5 to about 10, about 6 to about 10, about 7 to about 10. about 8 to about 10, about 2 to about 9, about 3 to about 9, about 4 to about 9, about 5 to about 9, about 6 to about 9, about 7 to about 9, about 8 to about 9, about 2 to about 8, about 3 to about 8, about 4 to about 8, about 5 to about 8, about 6 to about 8, about 7 to about 8, about 2 to about 7, about 3 to about 7, about 4 to about 7, about 5 to about 7, or about 6 to about 7; and
surface areas of at least one of the first and second working electrodes is from about 1.0 cm² to 5.0 cm², about 1.5 cm² to 5.0 cm², about 1.5 cm² to 4.5 cm², about 1.5 cm² to 4.0 cm², about 1.5 cm² to 3.9 cm², about 1.7 cm² to 3.8 cm², or about 1.7 cm² to 3.7 cm², about 1.7 cm² to 3.6 cm², about 1.7 cm² to 3.5 cm², about 1.7 cm² to 3.4 cm², about 1.7 cm² to 3.3 cm², or about 1.7 cm² to 3.2 cm².

5. The biosensor of any one of claims 1 to 4, wherein the first working electrode (E1) is configured to provide a decrease in the signal and the second working electrode (E2) is configured to provide an increase in the signal in the presence of the analyte; optionally, the decrease in the signal is about 0.1 to about 0.6 fold decrease and the increase in the signal is about 50 to about 200 fold increase.

6. The biosensor of any one of claims 1 to 5, wherein the biosensor is an electrochemical chip (20).

7. The biosensor of any one of claims 1 to 6, wherein the reporter moiety comprises a moiety with a redox, photoelectrochemical, semi-conductive and/or conductive species; optionally, the reporter moiety comprises a redox species; optionally, wherein one or more of:
the reporter moiety comprises a biopolymer modified with the redox species;
the redox species is selected from methylene blue, methylene blue succinymide, methylene blue maleimide, Atto MB2 maleimide (Sigma Aldrich) and other methylene blue derivatives; 3,7-Bis-[(2-Ammoniumethyl) (methyl)amino]phenothiazin-5-ium trifluoroacetate; 3,7-Bis-(piperazin-4-ium-1-yl)phenothiazin-5-ium trifluoroacetate; 3,7-Bis-[(2-ammoniumethyl)(methyl)amino] phenothiazin-5-ium chloride; and 3,7-Bis-(piperazin-4-ium-1-yl)phenothiazin-5-ium chloride, and/or ferrocene; and
the redox species is methylene blue and/or ferrocene.

8. The biosensor of any one of claims 1 to 7, wherein one or more of:
the reporter moiety is for transducing the presence of an analyte recognized by the recognition moiety to a detectable signal;
the reporter moiety comprises an antibody, an antigen, oligonucleotide, oligopeptide, and/or oligosaccharide; and
the recognition moiety comprises a DNAzyme, aptamer, antibody, and/or nucleic acid that is able to recognize the presence of the analyte, optionally, the recognition moiety is a DNAzyme that cleaves RNA in the presence of the analyte, an aptamer that changes conformation in presence of the analyte, or an antibody, optionally, the recognition moiety is a DNAzyme that cleaves RNA in the presence of the analyte to release the reporter moiety from the first working electrode (E1).

9. The biosensor of any one of claims 1 to 8, wherein the capture probe (40) comprises ssDNA, ssPNA, dsDNA, ssRNA, and/or dsRNA; optionally, the capture probe comprises ssDNA.

10. The biosensor of any one of claims 1 to 9, wherein one or more of:
the analyte is a toxin, biopolymer, cell, tissue, and/or pathogen; and
the analyte is a microorganism and/or virus; optionally, wherein one or more of:
the microorganism is a gram-negative bacterium;
the microorganism is *Escherichia coli, Salmonella typhimurium, Pseudomonas peli, Brevundimonas diminuta, Hafnia alvei, Yersinia ruckeri, Ochrobactrum grignonese, Achromobacter xylosoxidans, Moraxella osloensis, Acinetobacter lwoffi,* or *Serratia fonticola*;
the microorganism is a gram-positive bacterium;
the microorganism is *Listeria monocytogenes, Bacillus subtilis, Clostridium difficile, Actinomyces orientalis, Pediococcus acidilactici, Leuconostoc mesenteroides, Lactobacillus planturum,* or a combination thereof; and
the microorganism is a pathogenic bacterium.

11. A device comprising the biosensor according to claims 1 to 10; optionally, wherein one or more of:
the device is used for clinical and agricultural diagnostics, agri-food quality control, environmental monitoring, health monitoring, and/or pharmaceutical development;
the device is used for screening; and
the device is a hand-held device.

12. A kit for detection of an analyte comprising a biosensor of any one of claims 1 to 10 or device of claim 11 and instructions for use.

13. A method of determining the presence of an analyte in a sample comprising:
exposing the biosensor of any one of claims 1 to 10 to the sample to delocalize the reporter moiety from the first working electrode to the second working electrode in the presence of the analyte; and
measuring a signal at each of the first and second working electrodes to determine if there has been a change in signal at each of the first working electrode and the second working electrode indicating the presence of the analyte.

14. The method of claim 13, wherein one or more of:
the biosensor is exposed to the sample under conditions to delocalize the reporter moiety from the first working electrode to the second working electrode;
the change in signal from the first working electrode is a decrease in signal and the change in signal from the second working electrode is an increase in signal;
prior to exposing the biosensor to the sample, an initial signal is measured at each of the first and second working electrodes and after exposing the biosensor to the analyte, a final signal is measured at each of the first and second working electrodes, whereby differential change in signal between the initial signal and the final signal of the first working electrode and a change in signal between the initial signal and the final signal of the second working electrode, indicates the presence of the analyte in the sample;
the change in signal for the first working electrode and/or the second working electrode comprises a change in current, potential or impedance; optionally, the change in signal for the first working electrode and/or the second working electrode is a change in current; and
the analyte is a toxin, biopolymer, cell, tissue, and/or pathogen; optionally, wherein:
i) the analyte is a microorganism and/or virus; optionally, wherein:
the microorganism is a gram-negative bacterium;
the microorganism is *Escherichia coli, Salmonella typhimurium, Pseudomonas peli, Brevundimonas diminuta, Hafnia alvei, Yersinia ruckeri, Ochrobactrum grignonese, Achromobacter xylosoxidans, Moraxella osloensis, Acinetobacter lwoffi,* or *Serratia fonticola*;
the microorganism is a gram-positive bacterium;
the microorganism is *Listeria monocytogenes, Bacillus subtilis,*
*Clostridium difficile, Actinomyces orientalis, Pediococcus acidilactici, Leuconostoc mesenteroides, Lactobacillus planturum,* or a combination thereof; or
the microorganism is a pathogenic bacterium; or
ii) the analyte is the pathogen and the pathogen is *E. coli.*

15. The method of claim 13 or 14, wherein exposing the biosensor to the sample comprises contacting the biosensor with the sample and/or the sample is a urine or blood sample.

## Patentansprüche

1. Biosensor (10) zum Nachweis eines Analyten (36), umfassend:
a) eine erste (E1) und eine zweite (E2) Arbeitselektrode;
b) eine auf der ersten Arbeitselektrode funktionalisierte Detektionssonde (30), die eine Reportereinheit (32) umfasst, die an eine Erkennungseinheit (34) für einen Analyten gekoppelt ist, wobei die Detektionssonde so ausgelegt ist, dass sie die Reportereinheit von der ersten Arbeitselektrode zur zweiten Arbeitselektrode delokalisiert, wenn der Analyt an die Erkennungseinheit koppelt;
c) eine auf der zweiten Arbeitselektrode funktionalisierte Fängersonde (40), die die Reportereinheit erkennt und an sie koppelt; und
d) eine Gegenelektrode (24);
wobei jede Arbeitselektrode so konfiguriert ist, dass sie bei Vorhandensein des Analyten eine Signaländerung erzeugt.

2. Biosensor nach Anspruch 1, wobei:
der Biosensor ferner eine Referenzelektrode (22) umfasst; oder
die Gegenelektrode sowohl Gegenelektrode als auch Referenzelektrode ist.

3. Biosensor nach Anspruch 1 oder 2, wobei eines oder mehrere von:
der ersten (E1) und der zweiten (E2) Arbeitselektrode konzentrische Spiralelektroden, Serpentinenlinienelektroden, Bürstenelektroden, verschlungene Sternelektroden und/oder mehrere verschlungene Elektroden sind;
der ersten und der zweiten Arbeitselektrode nahe beieinander liegen, sodass der Reporteranteil von der Detektionssonde zur Abfangsonde transportiert werden kann;
den Arbeitselektroden nebeneinander liegen; und
zwischen mindestens einem Teil der ersten Arbeitselektrode und mindestens einem Teil der zweiten Arbeitselektrode ein Spalt besteht, in dem die Reportereinheit von der Detektionssonde zur Einfangssonde transportieren kann; optional der Spalt etwa 10 µm bis etwa 2 mm, etwa 10 µm bis etwa 1 mm, etwa 10 µm bis etwa 500 µm, etwa 10 µm bis etwa 400 µm, etwa 10 µm bis etwa 300 µm, etwa 10 µm bis etwa 200 µm oder etwa 10 µm bis etwa 100 µm beträgt.

4. Biosensor nach einem der Ansprüche 1 bis 3, wobei eines oder mehrere von:
mindestens einer der ersten (E1) und zweiten (E2) Arbeitselektroden eine nanostrukturierte Elektrode ist;
mindestens einer der ersten und zweiten Arbeitselektroden Kanten aufweist; optional mindestens eine der ersten und zweiten Arbeitselektroden scharfe Kanten und/oder Spitzen aufweist;
mindestens eine der ersten und zweiten Arbeitselektroden ein hohes Aspektverhältnis aufweist;
optional das hohe Aspektverhältnis etwa 2 bis etwa 10, etwa 3 bis etwa 10, etwa 4 bis etwa 10, etwa 5 bis etwa 10, etwa 6 bis etwa 10, etwa 7 bis etwa 10, etwa 8 bis etwa 10, etwa 2 bis etwa 9, etwa 3 bis etwa 9, etwa 4 bis etwa 9, etwa 5 bis etwa 9, etwa 6 bis etwa 9, etwa 7 bis etwa 9, etwa 8 bis etwa 9, etwa 2 bis etwa 8, etwa 3 bis etwa 8, etwa 4 bis etwa 8, etwa 5 bis etwa 8, etwa 6 bis etwa 8, etwa 7 bis etwa 8, etwa 2 bis etwa 7, etwa 3 bis etwa 7, etwa 4 bis etwa 7, etwa 5 bis etwa 7 oder etwa 6 bis etwa 7 beträgt; und
die Oberflächenbereiche von mindestens einer der ersten und zweiten Arbeitselektroden etwa 1,0 cm² bis 5,0 cm², etwa 1,5 cm² bis 5,0 cm², etwa 1,5 cm² bis 4,5 cm², etwa 1,5 cm² bis 4,0 cm², etwa 1,5 cm² bis 3,9 cm², etwa 1,7 cm² bis 3,8 cm², etwa 1,7 cm² bis 3,7 cm², etwa 1,7 cm² bis 3,6 cm², etwa 1,7 cm² bis 3,5 cm², etwa 1,7 cm² bis 3,4 cm², etwa 1,7 cm² bis 3,3 cm² oder etwa 1,7 cm² bis 3,2 cm² betragen.

5. Biosensor nach einem der Ansprüche 1 bis 4, wobei die erste Arbeitselektrode (E1) so konfiguriert ist, dass sie eine Verringerung des Signals bewirkt, und die zweite Arbeitselektrode (E2) so konfiguriert ist, dass sie in Gegenwart des Analyten eine Erhöhung des Signals bewirkt;
optional die Abnahme des Signals etwa das 0,1- bis 0,6-Fache und die Zunahme des Signals etwa das 50- bis 200-Fache beträgt.

6. Biosensor nach einem der Ansprüche 1 bis 5, wobei der Biosensor ein elektrochemischer Chip (20) ist.

7. Biosensor nach einem der Ansprüche 1 bis 6, wobei die Reportereinheit eine Einheit mit einer Redox-, photoelektrochemischen, halbleitenden und/oder leitenden Spezies umfasst; optional die Reportereinheit eine Redoxspezies umfasst; optional wobei eines oder mehrere von Folgendem:
die Reportereinheit aus einem mit der Redoxspezies modifizierten Biopolymer besteht;
die Redoxspezies ausgewählt ist aus Methylenblau, Methylenblausuccinymid, Methylenblaumaleimid, Atto MB2 Maleimid (Sigma Aldrich) und anderen Methylenblauderivaten; 3,7-Bis-[(2-Ammoniumethyl)(methyl)amino]phenothiazin-5-iumtrifluoracetat; 3,7-Bis-(piperazin-4-ium-1-yl)phenothiazin-5-iumtrifluoracetat; 3,7-Bis-[(2-ammoniumethyl)(methyl)amino]phenothiazin-5-iumchlorid;
und 3,7-Bis-(piperazin-4-ium-1-yl)phenothiazin-5-iumchlorid und/oder Ferrocen; und
die Redoxspezies Methylenblau und/oder Ferrocen ist.

8. Biosensor nach einem der Ansprüche 1 bis 7, wobei eines oder mehrere von Folgendem zutreffen:
die Reportereinheit dazu dient, die Anwesenheit eines vom Erkennungselement erkannten Analyten in ein nachweisbares Signal umzuwandeln;
die Reportereinheit einen Antikörper, ein Antigen, ein Oligonukleotid, ein Oligopeptid und/oder ein Oligosaccharid umfasst; und
die Erkennungseinheit ein DNAzym, Aptamer, einen Antikörper und/oder eine Nukleinsäure umfasst, die die Anwesenheit des Analyten erkennen kann, optional die Erkennungseinheit ein DNAzym, das RNA in Gegenwart des Analyten spaltet, ein Aptamer, das in Gegenwart des Analyten seine Konformation ändert, oder ein Antikörper ist, optional die Erkennungseinheit ein DNAzym ist, das RNA in Gegenwart des Analyten spaltet, um die Reportereinheit von der ersten Arbeitselektrode (E1) freizusetzen.

9. Biosensor nach einem der Ansprüche 1 bis 8, wobei die Erfassungssonde (40) ssDNA, ssPNA, dsDNA, ssRNA und/oder dsRNA umfasst; optional die Erfassungssonde ssDNA umfasst.

10. Biosensor nach einem der Ansprüche 1 bis 9, wobei eines oder mehrere von Folgendem zutreffen:
der Analyt ein Toxin, ein Biopolymer, eine Zelle, ein Gewebe und/oder ein Pathogen ist; und
der Analyt ein Mikroorganismus und/oder ein Virus ist;
optional, wobei eines oder mehrere von Folgendem zutreffen:
der Mikroorganismus ein gramnegatives Bakterium ist;
der Mikroorganismus *Eschenchia coli, Salmonella tyohimurium, Pseudomonas peli, Brevundimonas diminuta, Hafnia alvei, Yersinia ruckeri, Ochrobactrum grignonese, Achromobacter xylosoxidans, Moraxella osloensis, Acinetobacter Iwoffi oder Serratia fonticola* ist;
der Mikroorganismus ein grampositives Bakterium ist;
der Mikroorganismus *Listeria monocytogenes, Bacillus subtilis, Clostridium difficile, Actinomyces orientalis, Pediococcus acidilactici, Leuconostoc mesenteroides, Lactobacillus planturum* oder eine Kombination davon ist; und
der Mikroorganismus ein pathogenes Bakterium ist.

11. Gerät, das den Biosensor nach den Ansprüchen 1 bis 10 umfasst, optional wobei eines oder mehrere von Folgendem zutreffen:
das Gerät für die klinische und landwirtschaftliche Diagnostik, die Qualitätskontrolle im Agrar- und Lebensmittelbereich, das Umweltmonitoring, das Gesundheitsmonitoring und/oder die Arzneimittelentwicklung verwendet wird;
das Gerät zum Screening dient; und
es sich um ein Handgerät handelt.

12. Kit zum Nachweis eines Analyten, umfassend einen Biosensor nach einem der Ansprüche 1 bis 10 oder ein Gerät nach Anspruch 11 sowie eine Gebrauchsanweisung.

13. Verfahren zur Bestimmung des Vorhandenseins eines Analyten in einer Probe, umfassend:
Einwirkenlassen des Biosensors nach einem der Ansprüche 1 bis 10 auf die Probe, um die Reportereinheit in Gegenwart des Analyten von der ersten Arbeitselektrode zur zweiten Arbeitselektrode zu delokalisieren; und
Messen eines Signals an der ersten und zweiten Arbeitselektrode, um festzustellen, ob eine Signaländerung an der ersten und zweiten Arbeitselektrode aufgetreten ist, die das Vorhandensein des Analyten anzeigt.

14. Verfahren nach Anspruch 13, wobei eines oder mehrere von Folgendem zutreffen:
der Biosensor der Probe unter Bedingungen ausgesetzt wird, die eine Delokalisierung der Reportereinheit von der ersten Arbeitselektrode zur zweiten Arbeitselektrode ermöglichen;
die Signaländerung der ersten Arbeitselektrode eine Signalabnahme und die Signaländerung der zweiten Arbeitselektrode eine Signalzunahme ist;
bevor der Biosensor der Probe ausgesetzt wird, an der ersten und der zweiten Arbeitselektrode jeweils ein Anfangssignal gemessen wird und nachdem der Biosensor dem Analyten ausgesetzt wird, an der ersten und der zweiten Arbeitselektrode jeweils ein Endsignal gemessen wird, wobei eine differenzielle Signaländerung zwischen dem Anfangssignal und dem Endsignal der ersten Arbeitselektrode und eine Signaländerung zwischen dem Anfangssignal und dem Endsignal der zweiten Arbeitselektrode das Vorhandensein des Analyten in der Probe anzeigt;
die Signaländerung für die erste Arbeitselektrode und/oder die zweite Arbeitselektrode eine Strom-, Potential- oder Impedanzänderung umfasst; optional die Signaländerung für die erste Arbeitselektrode und/oder die zweite Arbeitselektrode eine Stromänderung ist; und
der Analyt ein Toxin, ein Biopolymer, eine Zelle, ein Gewebe und/oder ein Pathogen ist; optional, wobei:
i) der Analyt ein Mikroorganismus und/oder ein Virus ist; optional, wobei:
der Mikroorganismus ein gramnegatives Bakterium ist;
der Mikroorganismus *Eschenchia coli, Salmonella tyohimurium, Pseudomonas peli, Brevundimonas diminuta, Hafnia alvei, Yersinia ruckeri, Ochrobactrum grignonese, Achromobacter xylosoxidans, Moraxella osloensis, Acinetobacter Iwoffi oder Serratia fonticola* ist;
der Mikroorganismus ein grampositives Bakterium ist;
der Mikroorganismus *Listeria monocytogenes, Bacillus subtilis, Clostridium difficile, Actinomyces orientalis, Pediococcus acidilactici, Leuconostoc mesenteroides, Lactobacillus planturum* oder eine Kombination davon ist; oder
der Mikroorganismus ein pathogenes Bakterium ist; oder
ii) der Analyt der Pathogen ist und der Pathogen E. coli ist.

15. Verfahren nach Anspruch 13 oder 14, wobei das Aussetzen des Biosensors gegenüber der Probe das Kontaktieren des Biosensors mit der Probe umfasst und/oder die Probe eine Urin- oder Blutprobe ist.

## Revendications

1. Biocapteur (10) pour détecter un analyte (36) comprenant :
a) une première (E1) et une seconde (E2) électrode de travail ;
b) une sonde de détection (30) fonctionnalisée sur la première électrode de travail, la sonde de détection comprenant une fraction rapporteur (32) couplée à une fraction de reconnaissance (34) pour un analyte, dans lequel la sonde de détection est adaptée pour délocaliser la fraction rapporteur de la première électrode de travail à la seconde électrode de travail lorsque l'analyte se couple à la fraction de reconnaissance ;
c) une sonde de capture (40) fonctionnalisée sur la seconde électrode de travail, dans lequel la sonde de capture est destinée à reconnaître et à se coupler à la fraction rapporteur ; et
d) une contre-électrode (24) ;
dans lequel chaque électrode de travail est configurée pour fournir un changement de signal si l'analyte est présent.

2. Biocapteur selon la revendication **1,** dans lequel :
le biocapteur comprend en outre une électrode de référence (22) ; ou
la contre-électrode est à la fois une contre-électrode et une électrode de référence.

3. Biocapteur selon la revendication 1 ou 2, dans lequel un ou plusieurs parmi :
les première (E1) et seconde (E2) électrodes de travail sont choisies parmi des électrodes en spirale concentriques, des électrodes en ligne serpentine, des électrodes en brosse, des électrodes en étoile entrelacées et/ou des électrodes multiples entrelacées ;
la première électrode de travail et la seconde électrode de travail sont à proximité l'une de l'autre de telle sorte que la fraction reporter est capable de transporter de la sonde de détection à la sonde de capture ;
les électrodes de travail sont adjacentes l'une à l'autre ; et
il existe un espace entre au moins une partie de la première électrode de travail et au moins une partie de la seconde électrode de travail, dans lequel la fraction reporter est capable de transporter de la sonde de détection à la sonde de capture ; éventuellement, l'espace est d'environ 10 µm à environ 2 mm, d'environ 10 µm à environ 1 mm, d'environ 10 µm à environ 500 µm, d'environ 10 µm à environ 400 µm, d'environ 10 µm à environ 300 µm, d'environ 10 µm à environ 200 µm, ou d'environ 10 µm à environ 100 µm.

4. Biocapteur selon l'une quelconque des revendications 1 à 3, dans lequel un ou plusieurs parmi :
au moins l'une des première (E1) et seconde (E2) électrodes de travail est une électrode nanostructurée ;
au moins une des première et seconde électrodes de travail présente des bords ; éventuellement, au moins une des première et seconde électrodes de travail présente des bords et/ou des pointes tranchants ;
au moins une des première et seconde électrodes de travail présente un rapport hauteur/largeur élevé ;
éventuellement, ce rapport est d'environ 2 à environ 10, d'environ 3 à environ 10, d'environ 4 à environ 10, d'environ 5 à environ 10, d'environ 6 à environ 10, d'environ 7 à environ 10, d'environ 8 à environ 10, d'environ 2 à environ 9, d'environ 3 à environ 9, d'environ 4 à environ 9, d'environ 5 à environ 9, d'environ 6 à environ 9, d'environ 7 à environ 9, d'environ 8 à environ 9, d'environ 2 à environ 8, d'environ 3 à environ 8, d'environ 4 à environ 8, d'environ 5 à environ 8, d'environ 6 à environ 8, d'environ 7 à environ 8, d'environ 2 à environ 7, d'environ 3 à environ 7, d'environ 4 à environ 7, d'environ 5 à environ 7, ou d'environ 6 à environ 7 ; et
les surfaces spécifiques d'au moins une des première et seconde électrodes de travail sont d'environ 1,0 cm² à 5,0 cm², d'environ 1,5 cm² à 5,0 cm², d'environ 1,5 cm² à 4,5 cm², d'environ 1,5 cm² à 4,0 cm², d'environ 1,5 cm² à 3,9 cm², d'environ 1,7 cm² à 3,8 cm², ou d'environ 1,7 cm² à 3,7 cm², d'environ 1,7 cm² à 3,6 cm², d'environ 1,7 cm² à 3,5 cm², d'environ 1,7 cm² à 3,4 cm², d'environ 1,7 cm² à 3,3 cm², ou d'environ 1,7 cm² à 3,2 cm².

5. Biocapteur selon l'une quelconque des revendications 1 à 4, dans lequel la première électrode de travail (E1) est configurée pour fournir une diminution du signal et la seconde électrode de travail (E2) est configurée pour fournir une augmentation du signal en présence de l'analyte ; éventuellement, la diminution du signal est une diminution d'environ 0,1 à environ 0,6 fois et l'augmentation du signal est une augmentation d'environ 50 à environ 200 fois.

6. Biocapteur selon l'une quelconque des revendications 1 à 5, dans lequel le biocapteur est une puce électrochimique (20).

7. Biocapteur selon l'une quelconque des revendications 1 à 6, dans lequel la fraction rapporteur comprend une fraction avec une espèce redox, photoélectrochimique, semi-conductrice et/ou conductrice ; éventuellement, la fraction rapporteur comprend une espèce redox ; éventuellement, dans lequel un ou plusieurs parmi :
la fraction rapporteur comprend un biopolymère modifié avec l'espèce redox ;
l'espèce redox est choisie parmi le bleu de méthylène, le succinymide de bleu de méthylène, le maléimide de bleu de méthylène, le maléimide Atto MB2 (Sigma Aldrich) et d'autres dérivés du bleu de méthylène ; le trifluoroacétate de 3,7-bis-[(2-ammoniuméthyl) (méthyl)amino]phénothiazin-5-ium; le trifluoroacétate de 3,7-bis-(pipérazin-4-ium-1-yl)phénothiazin-5-ium ; le chlorure de 3,7-bis-[(2-ammoniuméthyl) (méthyl)amino]phénothiazin-5-ium ; et le chlorure de 3,7-bis-(pipérazin-4-ium-1-yl)phénothiazin-5-ium, et/ou le ferrocène ; et
l'espèce redox est le bleu de méthylène et/ou le ferrocène.

8. Biocapteur selon l'une quelconque des revendications 1 à 7, dans lequel un ou plusieurs parmi :
la fraction rapporteur sert à transduire la présence d'un analyte reconnu par la fraction de reconnaissance en un signal détectable ;
la fraction rapporteur comprend un anticorps, un antigène, un oligonucléotide, un oligopeptide et/ou un oligosaccharide ; et
la fraction de reconnaissance comprend un ADNzyme, un aptamère, un anticorps et/ou un acide nucléique capable de reconnaître la présence de l'analyte, éventuellement, la fraction de reconnaissance est un ADNzyme qui clive l'ARN en présence de l'analyte, un aptamère qui change de conformation en présence de l'analyte, ou un anticorps, éventuellement, la fraction de reconnaissance est un ADNzyme qui clive l'ARN en présence de l'analyte pour libérer la fraction rapporteur de la première électrode de travail (E1).

9. Biocapteur selon l'une quelconque des revendications 1 à 8, dans lequel la sonde de capture (40) comprend de l'ADNss, de l'APNss, de l'ADNdb, de l'ARNss et/ou de l'ARNdb ; éventuellement, la sonde de capture comprend de l'ADNss.

10. Biocapteur selon l'une quelconque des revendications 1 à **9,** dans lequel un ou plusieurs parmi :
l'analyte est une toxine, un biopolymère, une cellule, un tissu et/ou un pathogène ; et
l'analyte est un micro-organisme et/ou un virus ; éventuellement, dans lequel un ou plusieurs parmi :
le micro-organisme est une bactérie à Gram négatif ;
le micro-organisme est *Escherichia coli, Salmonella typhimurium, Pseudomonas peli, Brevundimonas diminuta, Hafnia alvei, Yersinia ruckeri, Ochrobactrum grignonese, Achromobacterxylosoxidans, Moraxella osloensis, Acinetobacter lwoffi,* ou *Serratia fonticola ;*
le micro-organisme est une bactérie à Gram positif ;
le micro-organisme est *Listeria monocytogenes, Bacillus subtilis, Clostridium difficile, Actinomyces orientalis, Pediococcus acidilactici, Leuconostoc mesenteroides, Lactobacillus planturum,* ou une combinaison de ceux-ci ;
et le micro-organisme est une bactérie pathogène.

11. Dispositif comprenant le biocapteur selon les revendications 1 à 10 ; éventuellement, dans lequel un ou plusieurs parmi :
le dispositif est utilisé pour le diagnostic clinique et agricole, le contrôle de la qualité agroalimentaire, la surveillance environnementale, la surveillance sanitaire, et/ou le développement pharmaceutique ; le dispositif est utilisé pour le dépistage ; et
le dispositif est un dispositif portatif.

12. Kit de détection d'un analyte comprenant un biocapteur selon l'une quelconque des revendications 1 à 10 ou un dispositif selon la revendication 11 et des instructions d'utilisation.

13. Procédé de détermination de la présence d'un analyte dans un échantillon, comprenant :
l'exposition du biocapteur selon l'une quelconque des revendications 1 à 10 à l'échantillon pour délocaliser la fraction rapporteur de la première électrode de travail à la seconde électrode de travail en présence de l'analyte ; et
la mesure d'un signal à chacune des première et seconde électrodes de travail pour déterminer **s'il** y a eu un changement de signal à chacune des première et seconde électrodes de travail indiquant la présence de l'analyte.

14. Procédé selon la revendication 13, dans lequel un ou plusieurs parmi :
le biocapteur est exposé à l'échantillon dans des conditions permettant de délocaliser la fraction reporter de la première électrode de travail à la seconde électrode de travail ;
le changement de signal de la première électrode de travail est une diminution du signal et le changement de signal de la seconde électrode de travail est une augmentation du signal ;
avant d'exposer le biocapteur à l'échantillon, un signal initial est mesuré à chacune des première et seconde électrodes de travail et, après avoir exposé le biocapteur à l'analyte, un signal final est mesuré à chacune des première et seconde électrodes de travail, moyennant quoi un changement différentiel de signal entre le signal initial et le signal final de la première électrode de travail et un changement de signal entre le signal initial et le signal final de la seconde électrode de travail, indiquent la présence de l'analyte dans l'échantillon ;
le changement de signal pour la première électrode de travail et/ou la seconde électrode de travail comprend un changement de courant, de potentiel ou d'impédance ;
éventuellement, le changement de signal pour la première électrode de travail et/ou la seconde électrode de travail est un changement de courant ; et
l'analyte est une toxine, un biopolymère, une cellule, un tissu, et/ou un pathogène ; éventuellement, dans lequel :
i) l'analyte est un micro-organisme et/ou un virus ; éventuellement, dans lequel :
le micro-organisme est une bactérie à Gram négatif ;
le micro-organisme est *Escherichia coli, Salmonella typhimurium, Pseudomonas peli, Brevundimonas diminuta, Hafnia alvei, Yersinia ruckeri, Ochrobactrum grignonese, Achromobacter xylosoxidans, Moraxella osloensis, Acinetobacter lwoffi,* ou *Serratia fonticola ;*
le micro-organisme est une bactérie à Gram positif ;
le micro-organisme est *Listeria monocytogenes, Bacillus subtilis, Clostridium difficile, Actinomyces orientalis, Pediococcus acidilactici, Leuconostoc mesenteroides, Lactobacillus planturum,* ou une combinaison de ceux-ci ; ou
le micro-organisme est une bactérie pathogène ; ou
ii) l'analyte est le pathogène et le pathogène est E. *coli.*

15. Procédé selon la revendication 13 ou 14, dans lequel l'exposition du biocapteur à l'échantillon comprend la mise en contact du biocapteur avec l'échantillon et/ou l'échantillon est un échantillon d'urine ou de sang.
